# EUROPÄISCHE PATENTANMELDUNG

(11) **EP 1 637 602 A1**
(43) Veröffentlichungstag der Anmeldung: **22.03.2006**
(21) Anmeldenummer: 05015686.8
(22) Anmeldetag: 02.10.1997
(51) Int. Cl.: C12N 15/30, C07K 14/445, C12N 15/62, A61K 39/015, A61K 31/70, C07H 21/00, C12N 1/21, C12N 5/10, C12N 1/11, C12N 15/67

(54) **Rekombinantes Herstellungsverfahren für ein vollständiges Malaria-Antigen gp190/MSP1**

(30) Priorität: 02.10.1996 DE 19640817
(62) Teilanmeldung aus: 97911173.9
(71) Anmelder: Bujard, Hermann, 69120 Heidelberg (DE)
(72) Erfinder: Tolle, Ralf, Dr., 69190 Walldorf (DE); Pan, Weiqing, Dr. Second Military Med. University, Shanghai 200433 (CN)
(74) Vertreter: Grünecker, Kinkeldey, Stockmair & Schwanhäusser Anwaltssozietät

(57) **Zusammenfassung**

Die Erfindung betrifft ein rekombinantes Herstellungsverfahren für das komplette gp190/MSP1-Oberflächenprotein von Plasmodium, insbesondere Plasmodium falicparum, sowie die vollständige DNA-Sequenz dieses Proteins und geeignete Wirtsorganismen für die Expression der Sequenz, wodurch das Protein in seiner Gesamtheit erstmals außerhalb des Parasiten synthetisiert werden konnte.

Die Erfindung eröffnet erstmals die Möglichkeit, das gp190/MSP1-Oberflächenprotein in ausreichender Menge herzustellen; ferner ist es ein Gegenstand der Erfindung gp190/MSP1 als Impfstoff zur Verfügung zu stellen.

Schließlich gibt die Erfindung ein Verfahren zur Stabilisierung von AT-reichen Genen an (Abb. 1).

SEQUENCE LISTING

<110> Bujard, Hermann

<120> Method for producing recombinants intended for use in a
      complete Malaria antigene GP190/MSP1

<130> EP16795IFZpau

<140> EP 05 015 686.8
<141> 2005-07-19

<150> EP 97 911 173.9
<151> 1999-04-01

<150> PCT/EP97/05441
<151> 1997-10-02

<150> DE19640817.2
<151> 1996-10-02

<160> 3

<170> PatentIn Ver. 2.1

<210> 1
<211> 4920
<212> DNA
<213> Plasmodium falciparum

<400> 1
atgaagatca tattcttttt atgttcattt ctttttttta ttataaatac acaatgtgta 60
acacatgaaa gttatcaaga acttgtcaaa aaactagaag ctttagaaga tgcagtattg 120
acaggttata gtttatttca aaaggaaaaa atggtattaa atgaaggaac aagtggaaca 180
gctgttacaa ctagtacacc tggttcaaag ggttcagttg cttcaggtgg ttcaggtggc 240
tcagttgctt caggtggctc agttgcttca ggtggctcag ttgcttcagg tggctcagtt 300
gcttcaggtg gttcaggtaa ttcaagacgt acaaatcctt cagataattc aagtgattca 360
gatgctaaat cttacgctga tttaaaacac agagtacgaa attacttgtt aactatcaaa 420
gaactcaaat atcctcaact ctttgattta actaatcata tgttaacttt gtgtgataat 480
attcatggtt tcaaatattt aattgatgga tatgaagaaa ttaatgaatt attatataaa 540
ttaaactttt attttgattt attaagagca aaattaaatg atgtatgtgc taatgattat 600
tgtcaaatac ctttcaatct taaaattcgt gcaaatgaat tagacgtact taaaaaactt 660
gtgttcggat atagaaaacc attagacaat attaaagata atgtaggaaa aatggaagat 720
tacattaaaa aaaataaaaa aaccatagaa aatataaatg aattaattga agaaagtaag 780
aaaacaattg ataaaaataa gaatgcaact aaagaagaag aaaaaaaaaa attataccaa 840
gctcaatatg atctttctat ttacaataaa caattagaag aagcacataa tttaataagc 900
gttttagaaa aacgtattga cactttaaaa aaaaatgaaa acattaagga attacttgat 960
aagataaatg aaattaaaaa tcccccaccg gccaattctg gaaatacacc aaatactctc 1020
cttgataaga acaaaaaaat cgaggaacac gaaaaagaaa taaaagaaat tgccaaaact 1080
attaaattta atattgatag tttatttact gatccacttg aattagaata ctatttaaga 1140
gaaaaaaata aaaatattga tataagtgca aaggttgaaa caaaggaatc aactgaaccc 1200
aatgaatatc caaatggagt tacttatcct ttgtcatata acgatattaa caatgcttta 1260
aatgaactta attcttttgg tgatttaatt aatccatttg attatacaaa agaaccaagt 1320
aaaaacatat atactgataa tgaaagaaaa aaattcataa atgaaattaa ggaaaaaatt 1380
aaaatagaaa aaaaaaaaat tgaatctgat aaaaaatctt acgaagacag atctaagtct 1440
ttaaatgata taacaaaaga atatgaaaaa ttacttaatg aaatttatga tagcaaattc 1500
aataataata tagatttaac taatttcgaa aaaatgatgg gtaaaagata ttcatataaa 1560
gttgagaaac ttacacacca taatactttt gcatcctatg aaaattctaa acataatctt 1620
gaaaagttaa caaaagctct taaatatatg gaagattatt ctttaaggaa tatagtagtt 1680
gaaaaagaat taaaatatta taaaaattta ataagcaaaa tagaaaatga gattgaaaca 1740
ttagttgaaa atattaaaaa agatgaagaa cagctttttg aaaaaaaaat tactaaagac 1800
gaaaataaac cagatgaaaa aattttagaa gtatctgaca ttgtaaaagt acaagttcaa 1860
aaagttttat taatgaacaa aattgacgaa ttaaaaaaga ctcaattgat tttaaaaaat 1920
gtagaattaa aacataatat acatgttccc aattcttaca aacaagaaaa taagcaagaa 1980
ccttattatt taattgtgtt gaaaaaagaa attgataaat taaaagtgtt catgcctaag 2040
gtagaatcat tgataaatga agaaaaaaaa aacataaaaa cagaaggtca atcggataat 2100
tcggaaccat caaccgaagg agaaataaca ggacaagcaa ctacaaaacc tggacaacaa 2160
gcaggatctg ctttagaagg agattcagta caagcacaag cacaagaaca aaaacaagca 2220
caaccaccag taccagtacc agtaccagaa gcaaaagcac aagtcccaac accaccagca 2280

ccagtaaata ataaaactga aaatgtttcc aaattagatt atcttgaaaa attatatgaa 2340
tttttaaata cttcatatat atgtcacaaa tatattttgg tttcacactc aactatgaac 2400
gaaaagatat taaaacaata taaaattaca aaggaggaag aaagcaaatt aagttcatgt 2460
gatccattag acttattgtt taatatacaa aataacatac ctgtaatgta ttctatgttt 2520
gatagcttaa acaatagttt atcacaacta tttatggaaa tttatgaaaa agaaatggtt 2580
tgtaatttat ataaacttaa ggataatgac aaaattaaaa atttattaga ggaagcgaaa 2640
aaagtatcca catctgtaaa aactctttca agttcatcaa tgcaaccatt atcattaaca 2700
cctcaggata aacccgaagt aagtgcaaat gatgatacat cacattctac aaatttgaat 2760
aatagtttaa aattatttga aaacatattg agtcttggaa aaaacaaaaa tatataccaa 2820
gaattaatag gtcaaaaaag tagtgaaaac ttttatgaaa agatattaaa agatagtgat 2880
acattttata atgaatcttt tacaaatttt gtaaaatcta aagctgatga tattaattca 2940
ttgaatgatg aatcaaaaag gaagaaatta gaagaagata ttaataaatt aaaaaaaact 3000
ttacagttat catttgattt atataataaa tataaattaa aattagaaag attatttgat 3060
aaaaagaaaa cagttggtaa atataaaatg caaattaaaa aacttacttt attaaaagaa 3120
caattagaat caaaattgaa ttcacttaat aacccaaagc atgtattaca aaacttttct 3180
gtttctttta acaaaaaaaa agaagctgaa atagcagaaa ctgaaaacac attagaaaac 3240
acaaaaatat tattgaaaca ttataaagga cttgttaaat attataatgg tgaatcatct 3300
ccattaaaaa ctttaagtga agaatcaatt caaacagaag ataattatgc cagtttagaa 3360
aactttaaag tattaagtaa attagaagga aaattaaagg ataatttaaa tttagaaaag 3420
aaaaaattat catacttatc aagtggatta catcatttaa ttgctgaatt aaaagaagta 3480
ataaaaaata aaaattatac aggtaattct ccaagtgaaa ataatacgga tgttaacaat 3540
gcattagaat cttacaaaaa atttctccca gaaggaacag atgttgcaac agttgtaagt 3600
gaaagtggat ccgacacatt agaacaaagt caaccaaaga aaccagcatc aactcatgta 3660
ggagcagagt ctaacacaat aacaacatca caaaatgtcg atgatgaagt agatgacgta 3720
atcatagtac ctatatttgg agaatccgaa gaagattatg atgatttagg acaagtagta 3780
acaggagaag cagtaactcc ttccgtaatt gataacatac tttctaaaat tgaaaatgaa 3840
tatgaggttt tatatttaaa acctttagca ggtgtttata gaagtttaaa aaaacaatta 3900
gaaaataacg ttatgacatt taatgttaat gttaaggata ttttaaattc acgatttaat 3960
aaacgtgaaa atttcaaaaa tgttttagaa tcagatttaa ttccatataa agatttaaca 4020
tcaagtaatt atgttgtcaa agatccatat aaatttctta ataaagaaaa aagagataaa 4080
ttcttaagca gttataatta tattaaggat tcaatagata cggatataaa ttttgcaaat 4140
gatgttcttg gatattataa aatattatcc gaaaaatata aatcagattt agattcaatt 4200
aaaaaatata tcaacgacaa acaaggtgaa aatgagaaat accttccctt tttaaacaat 4260
attgagacct tatataaaac agttaatgat aaaattgatt tatttgtaat tcatttagaa 4320
gcaaaagttc taaattatac atatgagaaa tcaaacgtag aagttaaaat aaaagaactt 4380
aattacttaa aaacaattca agacaaattg gcagatttta aaaaaaataa caatttcgtt 4440
ggaattgctg atttatcaac agattataac cataataact tattgacaaa gttccttagt 4500
acaggtatgg tttttgaaaa tcttgctaaa accgttttat ctaatttact tgatggaaac 4560
ttgcaaggta tgttaaacat ttcacaacac caatgcgtaa aaaaacaatg tccacaaaat 4620
tctggatgtt tcagacattt agatgaaaga gaagaatgta aatgtttatt aaattacaaa 4680
caagaaggtg ataaatgtgt tgaaaatcca aatcctactt gtaacgaaaa taatggtgga 4740
tgtgatgcag atgccaaatg taccgaagaa gattcaggta gcaacggaaa gaaaatcaca 4800
tgtgaatgta ctaaacctga ttcttatcca cttttcgatg gtatttctgg cagttcctct 4860
aacttcttag gaatatcatt cttattaata ctcatgttaa tattatacag tttcatttaa 4920

<210> 2
<211> 4940
<212> DNA
<213> Plasmodium falciparum

<400> 2
cgcacgcgta tgaaaatcat tttcttcctc tgttcatttc tgttttttat catcaatact 60
cagtgcgtga cccacgaatc ctatcaggag ctggttaaga aactggaagc tttggaagat 120
gccgtcctta ccggatacag cctgttccag aaggagaaga tggtgctgaa tgaagggacg 180
agtggcacgg ccgttacaac cagcacaccc ggttctaaag ggtctgtggc tagcggtggc 240
tccggtgggt ctgtggcctc tgggggttcc gtcgcctccg gcggcagcgt ggcatcaggt 300
ggctcagtgg caagcggcgg ttccgggaac agtcgaagaa ccaatccatc tgacaactct 360
agcgattccg acgccaagtc ctacgccgac ctcaagcacc gagtgagaaa ctatctcctc 420
actatcaagg agctgaagta cccacagttg ttcgacctca ctaatcatat gctgacactg 480
tgtgataaca ttcatggctt caaatatctg attgacggtt acgaagagat caatgaactc 540
ctgtacaagt tgaatttcta cttcgacttg ctaagggcca aactgaatga cgtttgcgcc 600
aatgactatt gtcaaattcc attcaatttg aagatcagag ccaacgagtt ggacgtattg 660
aagaagttgg tcttcggata tcgcaagcct ctcgacaaca tcaaggacaa tgtgggaaag 720
atggaagatt atattaaaaa gaataagaag accatcgaga acattaacga gctgatcgaa 780
gaatccaaaa agaccataga caaaaataag aatgcaacca aggaggaaga aaagaagaag 840
ttgtaccagg cccagtacga cctgtccatc tataacaaac agcttgaaga agcccataac 900
ctcatcagcg tactggagaa gcgcatagac accctcaaga agaatgaaaa tatcaaagaa 960
ctgctcgaca agattaatga aattaagaat cctccgccag ccaactctgg gaacacccct 1020
aacacgctgc tggacaagaa caagaagata gaggagcacg agaaagagat caaagagatc 1080

gccaaaacca ttaagttcaa catagattct ctctttactg atccccttga gctggagtac 1140
tacttgagag agaagaataa gaatatagac atctccgcca aagtcgagac aaaggaatca 1200
accgaaccta atgaatatcc caatggtgtg acgtaccctc tgtcttataa cgatatcaac 1260
aacgctctca acgagctcaa tagcttcggt gacttgatta accccttcga ttatacgaaa 1320
gaaccctcta agaatatcta cacagacaat gagagaaaga agtttatcaa cgaaatcaag 1380
gagaagatca aaattgagaa gaagaaaatt gagagtgaca agaaaagtta cgaagaccgc 1440
agcaaaagtc taaacgatat cactaaagag tatgaaaagc tgctgaacga gatctatgat 1500
tccaaattca acaataacat cgacctgacc aacttcgaga aaatgatggg aaaacggtac 1560
tcttacaaag tggagaaact gacacaccat aatacctttg catcctatga gaattctaag 1620
cataatcttg agaagctcac caaagctctt aagtatatgg aggactattc tctgcggaac 1680
attgttgtgg agaaagaact aaagtattac aagaatctca taagtaagat cgaaaacgag 1740
atcgagacgc ttgttgagaa cattaagaag gatgaagaac agttgtttga gaagaagatt 1800
acaaaagacg aaaataaacc agatgagaag atcctggagg tctccgatat tgttaaagtc 1860
caagtgcaga aggtgctcct catgaacaag attgatgaac tcaagaagac tcaactcatt 1920
ctgaagaacg tggagttaaa acataatata catgtgccga atagttataa gcaggagaat 1980
aagcaggaac catactacct catcgtactc aagaaagaga tagacaaact gaaagtgttc 2040
atgcccaaag tcgagagcct gatcaacgaa gagaagaaga acattaaaac tgaaggacag 2100
tcagataact ccgagccttc cacagaagga gagataaccg gacaggctac caccaagccc 2160
ggacaacagg ccggttcagc tctcgaaggc gatagcgtgc aagctcaagc acaagagcag 2220
aagcaggcac agcctccagt gccagtgccc gttccagagg ctaaagctca agtgcctaca 2280
ccaccagctc ctgtgaataa caagaccgag aatgtcagca aactggacta ccttgagaag 2340
ctctatgagt tcctgaatac atcctacatc tgccacaaat atatcctcgt ctctcacagc 2400
actatgaacg agaagattct taaacagtac aagataacca aggaagagga gagtaaactg 2460
tcctcttgtg atccactgga cctgctgttc aatatccaga acaacattcc cgttatgtat 2520
tctatgttcg atagcctcaa caattctctc tctcaactgt tcatggagat atatgagaag 2580
gagatggtct gcaacctgta taaactcaaa gacaacgaca agattaagaa ccttctggag 2640
gaagctaaga aggtctccac ctctgttaaa actctctctt ccagctccat gcaaccactg 2700
tctctcacac ctcaagacaa gcccgaagtg agcgctaacg acgacacctc tcactcgacc 2760
aaccttaata actcactgaa actgtttgag aacatcctgt ctctcggcaa gaataagaac 2820
atctaccaag aacttattgg acagaaatcg tccgagaact tctacgagaa gatactgaaa 2880
gacagcgaca cattctataa cgagagcttc actaacttcg tgaaatctaa agccgatgat 2940
atcaactctc ttaacgatga atctaaacgt aagaagctgg aagaggacat caataagctg 3000
aagaagacac tgcaactgag cttcgacctg tacaacaagt acaaactgaa actggagaga 3060
ctcttcgaca agaagaagac agtcggcaag tataagatgc agatcaagaa gttgactctg 3120
ctcaaggagc agcttgaaag caaactcaac tcactgaaca atccgaaaca cgtactgcag 3180
aacttctcag tgttcttcaa caagaagaag gaagccgaga tcgccgagac agagaacact 3240
ctggagaaca ccaagattct tctcaaacac tacaaaggcc tcgtcaagta ttataatggc 3300
gagtcttctc ctctgaagac tctctccgag gagagcatcc agaccgagga taactacgcc 3360
agcctcgaga acttcaaggt cctgtctaag ctcgaaggca agctgaagga caacctgaac 3420
ctggagaaga agaagctcag ctacctctct agcggactgc atcacctgat cgccgagctc 3480
aaggaagtca ttaagaacaa gaactacacc ggcaatagcc caagcgagaa taatacagac 3540
gtgaataacg cactggaatc ttacaagaag ttcctgcctg aaggaacaga tgtcgccact 3600
gtggtgtctg aatctggctc cgacacactg gagcagtctc aacctaagaa gcctgcatct 3660
actcatgtcg gagccgagtc caatacaatt accacatctc agaacgtcga cgatgaggtc 3720
gatgacgtca tcattgtgcc tatcttcggc gagagcgagg aggactacga tgacctcggc 3780
caggtggtca ccggtgaggc tgtcactcct tccgtgattg ataacattct gtccaaaatc 3840
gagaacgaat acgaagtgct ctatctgaaa cctctggcag gcgtctatag gtctctcaag 3900
aaacagctgg agaataacgt gatgaccttc aatgtcaacg tgaaggacat tctgaacagc 3960
cgctttaata agagagaaaa tttcaagaac gtcttggaga gcgacttgat tccctataaa 4020
gacctgacct cctctaacta cgttgtcaag gacccataca agttcctcaa taaagagaag 4080
agggataaat ttctgtctag ttacaactat atcaaggact ccatcgacac cgatatcaat 4140
ttcgctaatg atgtgctggg gtattacaag atcctgagcg aaaaatacaa gtctgacctt 4200
gactctatta aaaagtatat caacgataag caaggcgaga atgaaaaata tctgcccttc 4260
ctgaataaca tcgaaaccct gtacaagaca gtgaacgaca aaatcgacct cttcgtaatt 4320
cacctggagg ccaaggtcct caactatact tacgagaaga gcaatgtgga agttaaaatc 4380
aaggagctga actacctcaa aacaatccaa gacaagctgg cagatttcaa gaaaaataac 4440
aatttcgtcg gaattgcaga cctgtctacc gattataacc acaacaatct cctgaccaag 4500
tttctgtcca ctggcatggt gttcgaaaac ctcgccaaaa cagtgctgag caatctgctc 4560
gacggcaacc tgcagggcat gctgaacatc tcccagcacc aatgcgtgaa gaaacagtgc 4620
ccccagaata gcggctgttt caggcatctg gacgagcgcg aagagtgcaa gtgtctcctg 4680
aactacaaac aagaaggaga taagtgcgtg gagaacccaa accctacctg caatgaaaac 4740
aatggcgggt gtgacgccga tgctaaatgc accgaggaag acagcggctc taacggaaag 4800
aaaatcacat gcgagtgtac taagcccgac tcctatccac tcttcgacgg gattttttgc 4860
tccagctcta atttcctggg catctccttc ctgctgatcc tcatgctgat cctgtacagc 4920
ttcatctaat agatcgatgg 4940

<210> 3
<211> 1639
<212> PRT

<213> Plasmodium falciparum

<400> 3
Met Lys Ile Ile Phe Phe Leu Cys Ser Phe Leu Phe Phe Ile Ile Asn
1               5                   10                  15

Thr Gln Cys Val Thr His Glu Ser Tyr Gln Glu Leu Val Lys Lys Leu
            20                  25                  30

Glu Ala Leu Glu Asp Ala Val Leu Thr Gly Tyr Ser Leu Phe Gln Lys
        35                  40                  45

Glu Lys Met Val Leu Asn Glu Gly Thr Ser Gly Thr Ala Val Thr Thr
    50                  55                  60

Ser Thr Pro Gly Ser Lys Gly Ser Val Ala Ser Gly Gly Ser Gly Gly
65                  70                  75                  80

Ser Val Ala Ser Gly Gly Ser Val Ala Ser Gly Gly Ser Val Ala Ser
                85                  90                  95

Gly Gly Ser Val Ala Ser Gly Gly Ser Gly Asn Ser Arg Arg Thr Asn
            100                 105                 110

Pro Ser Asp Asn Ser Ser Asp Ser Asp Ala Lys Ser Tyr Ala Asp Leu
        115                 120                 125

Lys His Arg Val Arg Asn Tyr Leu Leu Thr Ile Lys Glu Leu Lys Tyr
    130                 135                 140

Pro Gln Leu Phe Asp Leu Thr Asn His Met Leu Thr Leu Cys Asp Asn
145                 150                 155                 160

Ile His Gly Phe Lys Tyr Leu Ile Asp Gly Tyr Glu Glu Ile Asn Glu
                165                 170                 175

Leu Leu Tyr Lys Leu Asn Phe Tyr Phe Asp Leu Leu Arg Ala Lys Leu
            180                 185                 190

Asn Asp Val Cys Ala Asn Asp Tyr Cys Gln Ile Pro Phe Asn Leu Lys
        195                 200                 205

Ile Arg Ala Asn Glu Leu Asp Val Leu Lys Lys Leu Val Phe Gly Tyr
    210                 215                 220

Arg Lys Pro Leu Asp Asn Ile Lys Asp Asn Val Gly Lys Met Glu Asp
225                 230                 235                 240

Tyr Ile Lys Lys Asn Lys Lys Thr Ile Glu Asn Ile Asn Glu Leu Ile
                245                 250                 255

Glu Glu Ser Lys Lys Thr Ile Asp Lys Asn Lys Asn Ala Thr Lys Glu
            260                 265                 270

Glu Glu Lys Lys Lys Leu Tyr Gln Ala Gln Tyr Asp Leu Ser Ile Tyr
        275                 280                 285

Asn Lys Gln Leu Glu Glu Ala His Asn Leu Ile Ser Val Leu Glu Lys
    290                 295                 300

Arg Ile Asp Thr Leu Lys Lys Asn Glu Asn Ile Lys Glu Leu Leu Asp
305                 310                 315                 320

Lys Ile Asn Glu Ile Lys Asn Pro Pro Pro Ala Asn Ser Gly Asn Thr
                325                 330                 335

Pro Asn Thr Leu Leu Asp Lys Asn Lys Lys Ile Glu Glu His Glu Lys
            340                 345                 350

Glu Ile Lys Glu Ile Ala Lys Thr Ile Lys Phe Asn Ile Asp Ser Leu
        355                 360                 365

Phe Thr Asp Pro Leu Glu Leu Glu Tyr Tyr Leu Arg Glu Lys Asn Lys
    370                 375                 380

Asn Ile Asp Ile Ser Ala Lys Val Glu Thr Lys Glu Ser Thr Glu Pro
385                 390                 395                 400

Asn Glu Tyr Pro Asn Gly Val Thr Tyr Pro Leu Ser Tyr Asn Asp Ile
                405                 410                 415

Asn Asn Ala Leu Asn Glu Leu Asn Ser Phe Gly Asp Leu Ile Asn Pro
            420                 425                 430

Phe Asp Tyr Thr Lys Glu Pro Ser Lys Asn Ile Tyr Thr Asp Asn Glu
        435                 440                 445

Arg Lys Lys Phe Ile Asn Glu Ile Lys Glu Lys Ile Lys Ile Glu Lys
    450                 455                 460

Lys Lys Ile Glu Ser Asp Lys Lys Ser Tyr Glu Asp Arg Ser Lys Ser
465                 470                 475                 480

Leu Asn Asp Ile Thr Lys Glu Tyr Glu Lys Leu Leu Asn Glu Ile Tyr
                485                 490                 495

Asp Ser Lys Phe Asn Asn Asn Ile Asp Leu Thr Asn Phe Glu Lys Met
            500                 505                 510

Met Gly Lys Arg Tyr Ser Tyr Lys Val Glu Lys Leu Thr His His Asn
        515                 520                 525

Thr Phe Ala Ser Tyr Glu Asn Ser Lys His Asn Leu Glu Lys Leu Thr
    530                 535                 540

Lys Ala Leu Lys Tyr Met Glu Asp Tyr Ser Leu Arg Asn Ile Val Val
545                 550                 555                 560

Glu Lys Glu Leu Lys Tyr Tyr Lys Asn Leu Ile Ser Lys Ile Glu Asn
                565                 570                 575

Glu Ile Glu Thr Leu Val Glu Asn Ile Lys Lys Asp Glu Glu Gln Leu
            580                 585                 590

Phe Glu Lys Lys Ile Thr Lys Asp Glu Asn Lys Pro Asp Glu Lys Ile
        595                 600                 605

Leu Glu Val Ser Asp Ile Val Lys Val Gln Val Gln Lys Val Leu Leu
    610                 615                 620

Met Asn Lys Ile Asp Glu Leu Lys Lys Thr Gln Leu Ile Leu Lys Asn
625                 630                 635                 640

Val Glu Leu Lys His Asn Ile His Val Pro Asn Ser Tyr Lys Gln Glu
                645                 650                 655

Asn Lys Gln Glu Pro Tyr Tyr Leu Ile Val Leu Lys Lys Glu Ile Asp
            660                 665                 670

Lys Leu Lys Val Phe Met Pro Lys Val Glu Ser Leu Ile Asn Glu Glu
        675                 680                 685

Lys Lys Asn Ile Lys Thr Glu Gly Gln Ser Asp Asn Ser Glu Pro Ser
    690                 695                 700

Thr Glu Gly Glu Ile Thr Gly Gln Ala Thr Thr Lys Pro Gly Gln Gln
705                 710                 715                 720

Ala Gly Ser Ala Leu Glu Gly Asp Ser Val Gln Ala Gln Ala Gln Glu
                725                 730                 735

Gln Lys Gln Ala Gln Pro Pro Val Pro Val Pro Val Pro Glu Ala Lys
            740                 745                 750

Ala Gln Val Pro Thr Pro Pro Ala Pro Val Asn Asn Lys Thr Glu Asn
        755                 760                 765

Val Ser Lys Leu Asp Tyr Leu Glu Lys Leu Tyr Glu Phe Leu Asn Thr
    770                 775                 780

Ser Tyr Ile Cys His Lys Tyr Ile Leu Val Ser His Ser Thr Met Asn
785                 790                 795                 800

Glu Lys Ile Leu Lys Gln Tyr Lys Ile Thr Lys Glu Glu Glu Ser Lys
                805                 810                 815

Leu Ser Ser Cys Asp Pro Leu Asp Leu Leu Phe Asn Ile Gln Asn Asn
            820                 825                 830

Ile Pro Val Met Tyr Ser Met Phe Asp Ser Leu Asn Asn Ser Leu Ser
        835                 840                 845

Gln Leu Phe Met Glu Ile Tyr Glu Lys Glu Met Val Cys Asn Leu Tyr
    850                 855                 860

Lys Leu Lys Asp Asn Asp Lys Ile Lys Asn Leu Leu Glu Glu Ala Lys
865                 870                 875                 880

Lys Val Ser Thr Ser Val Lys Thr Leu Ser Ser Ser Ser Met Gln Pro
                885                 890                 895

Leu Ser Leu Thr Pro Gln Asp Lys Pro Glu Val Ser Ala Asn Asp Asp
            900                 905                 910

Thr Ser His Ser Thr Asn Leu Asn Asn Ser Leu Lys Leu Phe Glu Asn
        915                 920                 925

Ile Leu Ser Leu Gly Lys Asn Lys Asn Ile Tyr Gln Glu Leu Ile Gly
    930                 935                 940

Gln Lys Ser Ser Glu Asn Phe Tyr Glu Lys Ile Leu Lys Asp Ser Asp
945                 950                 955                 960

Thr Phe Tyr Asn Glu Ser Phe Thr Asn Phe Val Lys Ser Lys Ala Asp
                965                 970                 975

Asp Ile Asn Ser Leu Asn Asp Glu Ser Lys Arg Lys Lys Leu Glu Glu
            980                 985                 990

Asp Ile Asn Lys Leu Lys Lys Thr Leu Gln Leu Ser Phe Asp Leu Tyr
        995                 1000                1005

Asn Lys Tyr Lys Leu Lys Leu Glu Arg Leu Phe Asp Lys Lys Lys Thr
    1010                1015                1020

Val Gly Lys Tyr Lys Met Gln Ile Lys Lys Leu Thr Leu Leu Lys Glu
1025                1030                1035                1040

Gln Leu Glu Ser Lys Leu Asn Ser Leu Asn Asn Pro Lys His Val Leu
                1045                1050                1055

Gln Asn Phe Ser Val Phe Phe Asn Lys Lys Lys Glu Ala Glu Ile Ala
            1060                1065                1070

Glu Thr Glu Asn Thr Leu Glu Asn Thr Lys Ile Leu Leu Lys His Tyr
        1075                1080                1085

Lys Gly Leu Val Lys Tyr Tyr Asn Gly Glu Ser Ser Pro Leu Lys Thr
1090                1095                1100

Leu Ser Glu Glu Ser Ile Gln Thr Glu Asp Asn Tyr Ala Ser Leu Glu
1105                1110                1115                1120

Asn Phe Lys Val Leu Ser Lys Leu Glu Gly Lys Leu Lys Asp Asn Leu
                1125                1130                1135

Asn Leu Glu Lys Lys Lys Leu Ser Tyr Leu Ser Ser Gly Leu His His
            1140                1145                1150

Leu Ile Ala Glu Leu Lys Glu Val Ile Lys Asn Lys Asn Tyr Thr Gly
        1155                1160                1165

Asn Ser Pro Ser Glu Asn Asn Thr Asp Val Asn Asn Ala Leu Glu Ser
    1170                1175                1180

Tyr Lys Lys Phe Leu Pro Glu Gly Thr Asp Val Ala Thr Val Val Ser
1185                1190                1195                1200

Glu Ser Gly Ser Asp Thr Leu Glu Gln Ser Gln Pro Lys Lys Pro Ala
                1205                1210                1215

Ser Thr His Val Gly Ala Glu Ser Asn Thr Ile Thr Thr Ser Gln Asn
            1220                1225                1230

Val Asp Asp Glu Val Asp Asp Val Ile Ile Val Pro Ile Phe Gly Glu
        1235                1240                1245

Ser Glu Glu Asp Tyr Asp Asp Leu Gly Gln Val Val Thr Gly Glu Ala
    1250                1255                1260

Val Thr Pro Ser Val Ile Asp Asn Ile Leu Ser Lys Ile Glu Asn Glu
1265                1270                1275                1280

Tyr Glu Val Leu Tyr Leu Lys Pro Leu Ala Gly Val Tyr Arg Ser Leu
                1285                1290                1295

Lys Lys Gln Leu Glu Asn Asn Val Met Thr Phe Asn Val Asn Val Lys
            1300                1305                1310

Asp Ile Leu Asn Ser Arg Phe Asn Lys Arg Glu Asn Phe Lys Asn Val
        1315                1320                1325

Leu Glu Ser Asp Leu Ile Pro Tyr Lys Asp Leu Thr Ser Ser Asn Tyr
    1330                1335                1340

Val Val Lys Asp Pro Tyr Lys Phe Leu Asn Lys Glu Lys Arg Asp Lys
1345                1350                1355                1360

Phe Leu Ser Ser Tyr Asn Tyr Ile Lys Asp Ser Ile Asp Thr Asp Ile
                1365                1370                1375

Asn Phe Ala Asn Asp Val Leu Gly Tyr Tyr Lys Ile Leu Ser Glu Lys
            1380                1385                1390

Tyr Lys Ser Asp Leu Asp Ser Ile Lys Lys Tyr Ile Asn Asp Lys Gln
        1395                1400                1405

Gly Glu Asn Glu Lys Tyr Leu Pro Phe Leu Asn Asn Ile Glu Thr Leu
    1410                1415                1420

Tyr Lys Thr Val Asn Asp Lys Ile Asp Leu Phe Val Ile His Leu Glu
1425                1430                1435                1440

Ala Lys Val Leu Asn Tyr Thr Tyr Glu Lys Ser Asn Val Glu Val Lys
                1445                1450                1455

Ile Lys Glu Leu Asn Tyr Leu Lys Thr Ile Gln Asp Lys Leu Ala Asp
            1460                1465                1470
Phe Lys Lys Asn Asn Asn Phe Val Gly Ile Ala Asp Leu Ser Thr Asp
        1475                1480                1485
Tyr Asn His Asn Asn Leu Leu Thr Lys Phe Leu Ser Thr Gly Met Val
    1490                1495                1500
Phe Glu Asn Leu Ala Lys Thr Val Leu Ser Asn Leu Leu Asp Gly Asn
1505                1510                1515                1520
Leu Gln Gly Met Leu Asn Ile Ser Gln His Gln Cys Val Lys Lys Gln
                1525                1530                1535
Cys Pro Gln Asn Ser Gly Cys Phe Arg His Leu Asp Glu Arg Glu Glu
            1540                1545                1550
Cys Lys Cys Leu Leu Asn Tyr Lys Gln Glu Gly Asp Lys Cys Val Glu
        1555                1560                1565
Asn Pro Asn Pro Thr Cys Asn Glu Asn Asn Gly Gly Cys Asp Ala Asp
    1570                1575                1580
Ala Lys Cys Thr Glu Glu Asp Ser Gly Ser Asn Gly Lys Lys Ile Thr
1585                1590                1595                1600
Cys Glu Cys Thr Lys Pro Asp Ser Tyr Pro Leu Phe Asp Gly Ile Phe
                1605                1610                1615
Cys Ser Ser Ser Asn Phe Leu Gly Ile Ser Phe Leu Leu Ile Leu Met
            1620                1625                1630
Leu Ile Leu Tyr Ser Phe Ile
        1635

## Beschreibung

Die Erfindung betrifft ein rekombinantes Herstellungsverfahren für das vollständige Malaria-Antigen gp190/MSP1 sowie einzelner natürlich vorkommender Domänen und Teile derselben durch Expression (einer) synthetischer DNA-Sequenzen. Die Erfindung betrifft außerdem die durch das Verfahren hergestellten DNA-Sequenzen und die für die Expression der DNA-Sequenzen verwendeten Wirtsorganismen. Ferner betrifft die Erfindung die Verwendung des vollständigen Malaria-Antigens sowie Teile derselben als Impfstoff zur Immunisierung gegen Malaria.

Schließlich betrifft die vorliegende Erfindung ein Stabilisierungsverfahren für AT-reiche Gene, sowie stabilisierte Gene, die sich durch einen geringeren AT-Gehalt auszeichnen.

Malaria ist weltweit eine der bedeutendsten Infektionskrankheiten. Nach Angaben der WHO waren 1990 in 99 Ländern 40% der Weltbevölkerung einem Malariarisiko ausgesetzt. Ihre Verbreitung nimmt derzeit wieder massiv zu. Dies ist vor allem auf eine intensive Resistenzbildung der Malariaerreger zurückzuführen, die dadurch gefördert wird, daß die zur Therapie eingesetzten Medikamente ebenfalls als Prophylaxe empfohlen und eingenommen werden. Neben der Suche nach neuen, wirksamen Chemotherapeutika werden heute Hoffnungen auch in die Entwicklung von Impfstoffen gesetzt, da Menschen in Malaria-epidemischen Regionen der Welt verschiedene Arten von Immunität zu entwickeln vermögen. Neben einer natürlichen Resistenz gegen Malaria, die sich bei heterozygoten Trägern des Sichelzellgens und bei Personen mit Thalassämie und Glucose-6-Phosphat-Dehydrogenase-Mangel ausbildet, können im Laufe einer Malariainfektion im Menschen Immunitätsmechanismen einsetzen, die sich in einer erhöhten Widerstandsfähigkeit gegenüber den Plasmodien äußern. Dementsprechend ist der Krankheitsverlauf in stark durchseuchten Bevölkerungspopulationen weitaus weniger bedrohlich als bei Personen, die der Infektion weniger häufig oder erstmalig ausgesetzt sind.

Das Hauptproblem bei der Impfstoffentwicklung ist die Identifikation eines Antigens, das schützende Immunität bewirken kann, da kein leicht zugängliches und gut definiertes Tiermodell für die vier den Menschen befallenden Parasiten vorhanden ist. Die Malaria-Erreger gehören der Gruppe der Plasmodien an, wobei die Infektion mit einem der vier Erreger Plasmodium vivax, Plasmodium ovale, Plasmodium malariae oder *Plasmodium falciparum* durch den Stich von Anopheles-Mücken erfolgt. Von diesem Erreger ist Plasmodium falciparum der gefährlichste und der am weitesten verbreitete.

Das Hauptoberflächenprotein von Merozoiten, der invasiven Form der Blutstadien des Malaria-Erregers Plasmodium falciparum und anderer Malaria-Erreger wie P. *vivax*, ist ein 190 - 220 kD Glykoprotein. Spät in der Entwicklung des Parasiten wird dieser Vorläufer in kleinere Proteine prozessiert, die jedoch als einheitlicher Komplex aus Merozoiten isoliert werden können. Der Komplex ist mittels eines Glycosylphosphatidyl-Inositol-Ankers mit der Merozoitenmembran verknüpft. Die Sequenzen der gp 190-Proteine verschiedener P. *falciparum-Stämme* fallen in zwei Gruppen, zwischen denen intragene Rekombination häufig ist. Insgesamt besteht das Protein aus mehreren hochkonservierten Regionen, aus einem dimorphem Bereich, welche jeweils einem von zwei Allelen angehören und aus zwei relativ kleinen oligomorphen Blöcken im N-terminalen Bereich (Tanabe, K., Mackay, M., Goman, M. und Scaife, J.G. (1987), Allelic dimorphism in a surface antigen gene of the malaria parasite *Plasmodium falciparum*. J. Mol. Biol. 195, 273-287; Miller, L.H., Roberts, T., Shahabuddin, M. und McCutchan, T.F. (1993), Analysis of sequence diversity in the Plasmodium falciparum merozoite surface protein-1 (MSP-1). Mol. Biochem. Parasitol. 59, 1-14).

Das gp190/MSP1 galt bereits früh als ein möglicher Kandidat für einen Impfstoff. So wurde im Nagermodell nach Immunisierung mit dem analogen Protein aktiver Schutz gegen Infektion mit Nager-Parasiten erhalten. Passiver Schutz ließ sich mit gegen dieses Protein gerichteten Antikörpern erreichen (siehe auch Holder, A. A. und Freeman, R.R. (1981), Immunization against blood-stage rodent malaria using purified parasite antigens, Nature 294, 361-364; Majarian, W.R., Daly, T. M., Weidanz, W.P. und Long, C.A. (1984), Passive immunization against murine malaria with an IgG3 monoclonal antibody, J. Immunol. 132, 3131-3137). Die Daten, die diese Annahme belegen sollen, sind im einzelnen jedoch statistisch nicht signifikant.

Darüber hinaus sind mehrere monoklonale Antikörper, welche in vitro die Invasion von Erythrozyten durch *P*. *falciparum* inhibieren, sind gegen gp190/MSP1 gerichtet (Pirson, P.J. und Perkins, M.E. (1985), Characterization with monoclonal antibodies of a surface antigen of *Plasmodium falciparum* merozoites. J. Immunol. 134, 1946-1951; Blackman, M.J., Heidrich, H.-G., Donachie, S., McBride, J.S. und Holder A.A. (1990), A single fragment of a malaria merozoite surface protein remains on the parasite during red cell invasion and is the target of invasion-inhibiting antibodies, J. Exp. Med. 172, 379-382).

Schließlich wurde eine Reihe von Impfstudien mit gp190/MSP1-Material aus *P. falciparum* an Primaten, insbesondere an Aotus und Saimiri-Affen durchgeführt (siehe auch Perrin, L.H., Merkli, B., Loche, M., Chizzolini, C., Smart, J. und Richle, R. (1984), Antimalarial immunity in Saimiri monkeys. Immunization with surface components of asexual blood stages, J. Exp. Med. 160, 441-451; Hall, R., Hyde, J.E., Goman, M., Simmons, D.L., Hope, I.A., Mackay, M. und Scaife, J.G. (1984), Major surface antigen gene of a human malaria parasite cloned and expressed in bacteria, Nature 311, 379-382; Siddiqui, W.A., Tam, L.Q., Kramer, K.J., Hui, G.S.N., Case, S.E., Yamaga, K.M., Chang, S.P., Chan, E.B.T. und Kan, S.-C. (1987), Merozoite surface coat precursor protein completely protects Aotus monkeys against *Plasmodium falciparum* malaria, Proc. Natl. Acad. Sci, USA 84, 3014-3018; Ettlinger, H.M., Caspers, P., Materile, H., Schoenfeld, H.-J., Stueber, D. und Takacs, B. (1991), Ability of recombinant or native proteins to protect monkeys against heterologous challenge with *Plasmodium falciparum*, Inf. Imm. 59, 3498-3503; Holder, A.A., Freeman, R.R. und Nicholls, S.C. (1988), Immunization against *Plasmodium falciparum* with recombinant polypeptides produced in *Escherichia coli*, Parasite Immunol. 10, 607-617; Herrera, S., Herrera, M.A., Perlaza, B.L., Burki, Y., Caspers, P., Döbeli, H., Rotmann, D. und Certa, U. (1990), Immunization of Aotus monkeys with *Plasmodium falciparum* blood-stage recombinant proteins, Proc. Natl. Acad. Sci. USA 87, 4017-4021; Herrera, M.A., Rosero, F., Herrera, S., Caspers, P., Rotmann, D., Sinigaglia,F. und Certa, U. (1992), Protection against malaria in Aotus monkeys immunized with a recombinant blood-stage antigen fused to a universal T-cell epitope; correlation of serum gamma interferon levels with protection, Inf. Imm. 60, 154-158; Patarroyo, M.E., Romero P., Torres, M.L., Clavijo, P., Moreno, A., Martinez, A., Rodriquez, R., Guzmann, F. und Cabezas, E. (1987), Induction of protective immunity against experimental infection with malaria using synthetic peptides, Nature 328, 629-632). In diesen Impfstudien lassen sich hierbei zwei Ansätze unterscheiden
- Verwendung von aus Parasiten isoliertem Material und
- Einsatz von in heterologen Expressionssystemen gewonnenem Material.

Letzteres bestand in der Regel aus relativ kleinen Teilbereichen des Gesamtproteins. Obwohl die ersten Resultate der in Vorversuchen an Affen durchgeführten Impfungen andeuten, daß gp190/MSP1 einen Schutz vermitteln könnte, haben alle an Primaten durchgeführten Experimente zwei Probleme, welche einen solchen Schluß in Frage stellen:
(a) sie wurden an zu kleinen Tiergruppen durchgeführt
(b) sie wurden nicht wiederholt.

Die Resultate und die daraus gezogenen Schlüsse sind daher statistisch nicht abgesichert. Neben dem schwierigen Zugang zu geeigneten Affen liegt das zugrunde liegende Hauptproblem darin, daß es bislang nicht möglich war, gutes Impfmaterial in ausreichender Menge herzustellen.

Andererseits konnten nach der Sequenzierung des gp190-Gens aus dem K1- und dem MAD20-Stamm von *Plasmodium falciparum* konnten überlappende Fragmente in *E. coli* exprimiert werden. Mit diesem Material zeigten epidemiologische Studien in Westafrika, daß in der Gruppe der Adoleszenten eine Korrelation bestand zwischen Antikörpertiter gegen gp190/MSP1-Fragmente einerseits und einem Schutz vor Parasiteninfektion andererseits. Darüber hinaus schien der Titer auch mit der Fähigkeit zu korrelieren, die Parasitämie auch auf niedrigem Niveau zu kontrollieren (Tolle et al. (1993): A prospective study of the association between the human humoral immune response to *Plasmodium falciparum* blood stage antigen gp190 and control of malarial infections. Infect Immun. 61, 40-47). Diese Resultate werden ergänzt durch neue Untersuchungen an Aotusaffen im Rahmen der vorliegenden Erfindung. Hier wurde ein hoher Schutz gegen Infektion mit dem Parasiten dadurch erreicht, daß Proteinpräparationen aus *Plasmodium falciparum,* die überwiegend aus nichtprozessiertem gp190/MSP1 bestanden, als Impfstoff benutzt worden waren. Die Affen mit dem höchsten Antikörpertiter gegen gp190/MSP1 waren am besten geschützt. Diese Resultate machten letztendlich das gp190 zu einem vielversprechenden Kandidaten für eine Impfstoff gegen Malaria tropica.

Von einigen Arbeitsgruppen wurde der C-terminalen Domäne des gp190 (p19 bzw. p42) eine besondere Rolle bei der gp190 vermittelten Immunität zugewiesen (siehe auch Chang, S.P., Case, S.E., Gosnell, W.L., Hashimoto, A., Kramer, K.J., Tam, L. Q., Hashiro, C.Q., Nikaido, C.M., Gibson, H.L., Lee-Ng, C.T., Barr, P.J., Yokota, B.T. und Hui, G.S.N.(1996), A recombinant baculovirus 42-kilodalton C-terminal fragment of *Plasmodium falciparum* merozoite surface protein 1 protects Aotus monkeys against malaria, Inf. Imm. 64, 253-261; Burghaus, P.A., Wellde, B.T., Hall, T., Richards, R.L., Egan, A.F., Riley, E.M., Ripley-Ballou, W. und Holder A.A. (1996), Immunization of Aotus nancymai with recombinant C-terminus of *Plasmodium falciparum* merozoite surface protein-1 in liposomes and alum adjuvant does not induce protection against a challenge infection, Inf. Imm., in press).

Bislang ist es jedoch nicht möglich, auf rationaler Basis andere Teile des gp190 als nicht relevant für eine schützende Immunantwort auszuschließen. Es ist daher nach wie vor notwendig, das gesamte Gen bzw. das intakte gp190 für Impfversuche zu verwenden. Trotz mehrfacher Versuche verschiedener Arbeitskreise ist es jedoch noch nicht gelungen, das gesamte Gen des gp190/MSP1 zu klonieren und zu exprimieren.

Bislang war es auch noch nicht möglich, a priori einen Teil aus der gp190-Sequenz für die schützende Immunantwort als nicht relevant auszuschließen, so daß es nach wie vor notwendig ist, das gesamte Gen bzw. das gesamte Genprodukt für Impfversuche zu verwenden. Es ist jedoch trotz vieler Versuche mehrerer Arbeitskreise noch nicht gelungen, das gesamte Gen des gp190/MSP1 zu klonieren.

Es war daher eine Aufgabe der vorliegenden Erfindung, Impfmaterial in Form von vollständigem gp190/MSP1 in ausreichender Menge zur Verfügung zu stellen. Es war eine weitere Aufgabe der vorliegenden Erfindung, ein Verfahren anzugeben, mit dem dieses Impfmaterial gewonnen werden kann.

Es war außerdem eine weitere Aufgabe gemäß der vorliegenden Erfindung, eine vollständige DNA-Sequenz von gp190/MSP1 anzugeben, die in einem Wirtsorganismus exprimierbar ist.

Weiterhin war es eine Aufgabe der vorliegenden Erfindung, Wirtsorganismen anzugeben, die das vollständige Gen gp190/MSP1 enthalten.

Schließlich war es auch eine Aufgabe der vorliegenden Erfindung, ein Stabilisierungsverfahren für AT-reiche Gene anzugeben, sowie ein für die Expression geeignetes, stabilisiertes Gen, das sich durch eine Erniedrigung des AT-Gehalts auszeichnet.

Diese Aufgaben werden durch die in den Ansprüchen angegebenen Gegenstände gelöst.

Im folgenden werden einige Begriffe näher erläutert, um klarzustellen, wie sie in diesem Zusammenhang verstanden werden sollen.

"Rekombinantes Herstellungsverfahren" bedeutet, daß ein Protein von einer DNA-Sequenz durch einen geeigneten Wirtsorganismus exprimiert wird, wobei die DNA-Sequenz aus einer Klonierung und Fusion einzelner DNA-Abschnitte entstanden ist.

"Vollständiges gp190/MSP1-Protein" meint hier das gesamte, aus o.g. Plasmodien, insbesondere *Plasmodium falciparum*, isolierbare gp190/MSP1-Oberflächenprotein, das das Hauptoberflächenprotein der Merozoiten des o.g. Erregers darstellt sowie die Proteine mit analoger Funktion aus den anderen Plasmodiumarten, wie *P*. *vivax*. Der Begriff betrifft somit jeweils das Hauptoberflächenprotein der Merozoiten der 4 o.g, für den Menschen gefährlichen Malariaerreger. "Vollständiges gp190/MSP1-Gen" ist das für dieses Protein kodierende Gen. "Vollständig" bedeutet in diesem Zusammenhang, daß die gesamte Aminosäuresequenz des nativen Proteins vorhanden ist, bzw. daß die Gensequenz für die gesamte Aminosäuresequenz des nativen Proteins kodiert.

Mit umfaßt sind jedoch auch mutierte und/oder verkürzte Formen von gp190/MSP1, sofern sie das gleiche Immunisierungspotential (Impfschutz) wie das vollständige gp190/MSP1 aufweisen. Schließlich umfaßt der Begriff auch Varianten von gp190/MSP1 die sich dadurch auszeichnen, daß sie Proteinabschnitte verschiedener Allele in einem Proteinmolekül enthalten.

"FCB-1" ist ein Stamm von *P. falciparum,* wie beschrieben bei Heidrich, H.-G., Miettinen-Baumann, A., Eckerskorn, C. und Lottspeich, F. (1989) The N-terminal amino acid sequences of the Plasmodium falciparum (FCB1) merozoite surface antigens of 42 and 36 kilodalton, both derived from the 185-195-kilodalton precursor. Mol. Biochem. Parasitol. 34, 147-154.

"Ankersignal" meint hier eine Proteinstruktur, für die eine DNA-Sequenz am 3'- oder 5'-Ende des erfindungsgemäßen Gens kodiert. Ankersignale sind Strukuren, die einem Polypeptid die Verankerung an anderen Strukturen, wie z.B. Membranen ermöglichen.

"Signalpeptid" bedeutet hier eine Proteinstruktur, für die eine DNA-Sequenz am N-terminalen Ende des erfindungsgemäßen Gens kodiert. Signalpeptide sind Strukturen, die dem Polypeptid u.a. ein Einschleusen in Membranen ermöglichen.

"AT-Gehalt" meint im Zusammenhang der vorliegenden Erfindung die prozentuale Menge von Adenin/Thymin-Basenpaaren im Verhältnis zu Guanin/Cytosin-Basenpaaren.

"Klonierung" soll hier alle im Stand der Technik bekannten Klonierungsmethoden umfassen, die hier zum Einsatz kommen könnten, die jedoch nicht alle im einzelnen beschrieben werden, weil sie zum selbstverständlichen Handwerkszeug des Fachmanns gehören.

"Expression in einem geeigneten Expressionssystem" soll hier alle im Stand der Technik bekannten Expressionsmethoden in bekannten Expressionssystemen umfassen, die hier zum Einsatz kommen könnten, die jedoch nicht alle im einzelnen beschrieben werden, weil sie zum selbstverständlichen Handwerkszeug des Fachmanns gehören.

Es ist eine erste Aufgabe gemäß der vorliegenden Erfindung, ein Verfahren anzugeben, durch das das gp190/MSP1-Protein und das Gen hierfür in ausreichender Menge ohne übermäßige Kosten produziert werden kann.

Diese Aufgabe wird durch das in Anspruch 1 beschriebene rekombinante Herstellungsverfahren gelöst, durch das ein voll-ständiges gp190/MSP-1-Gen und das davon kodierte Protein in ausreichender Menge erhältlich ist.

Durch dieses Verfahren wurde es erstmals möglich, das Protein in seiner Gesamtheit außerhalb des Parasiten zu synthetisieren. Das so synthetisierte Protein ist, wie die Analyse mit konformationelle Epitope erkennenden monoklonalen Antikörpern zeigt, zumindest über weite Bereiche in natürlich gefalteter Form herstellbar. Durch das rekombinante Herstellungsverfahren konnten jeweils mehrere Milligramm intaktes gp190/MSP1 aus den Wirtsorganismen gewonnen werden, eine Menge, die aus technischen und aus Kostengründen aus Parasiten praktisch nicht gewonnen werden kann. Die jetzt mögliche Produktion des Proteins in beliebigen Mengen eröffnet neue Perspektiven für seinen Einsatz als experimentellen Impfstoff gegen Malaria. Darüber hinaus ist der Weg frei für die Entwicklung von Lebendimpfstoffen sowie für Vakzine auf Nukleinsäure-Basis.

Vorzugsweise liegt der Synthese der für das Protein gp190/MSP1 kodierenden GenSequenz die Sequenz des *P. falciparum* FCB-1 Stammes zugrunde. *P. falciparum* ist der Erreger der Malaria tropica und damit der gefährlichste unter den Malaria-Arten. Das zugrunde liegende Gen ist ein Vertreter des "K1-Allels", wobei K1 für einen bestimmten *P. falciparum*-Stamm steht. Seine kodierende Sequenz erstreckt sich über 4917 Basenpaare und schließt eine Signalsequenz am N-terminalen Ende sowie eine Anker-Sequenz am C-terminalen Ende ein.

Weiterhin ist das rekombinante Herstellungsverfahren gemäß der Erfindung vorzugsweise dadurch gekennzeichnet, daß der AT-Gehalt der dem Protein zugrunde liegenden DNA-Sequenz gegenüber der Wildtyp-Sequenz erniedrigt ist, von 74% im ursprünglichen Gen auf vorzugsweise ca. 55%, indem bspw. unter Erhalt der Aminosäuresequenz des FCB-1-Proteins eine DNA-Sequenz mit den im menschlichen Genom üblichen Codon-Häufigkeiten hergestellt wird. Andere Codonhäufigkeiten, welche den AT-Gehalt erniedrigen, sind ebenfalls denkbar.

Vorzugsweise kodiert das dem durch das rekombinante Herstellungsverfahren erzeugte Protein zugrunde liegende Gen für die volle Aminosäuresequenz einschließlich Signalpeptid und GPI-Ankersignalpeptid, im weiteren als gp190^{s} bezeichnet.

In einer weiteren bevorzugten Ausführungform kodiert das dem durch das rekombinante Herstellungsverfahren erzeugte Protein zugrunde liegende Gen für die volle Aminosäuresequenz mit Ausnahme des GPI-Ankersignals. Diese Ausführungsform wird im weiteren als gp190^{S1} bezeichnet.

In noch einer weiteren bevorzugten Ausführung kodiert das dem durch das rekombinante Herstellungsverfahren erzeugte Protein zugrunde liegende Gen für die volle Aminosäuresequenz mit Ausnahme des GPI-Ankersignals und des Signalpeptids. Diese Ausführungsform wird im weiteren als gp190^{s2} bezeichnet.

In einer weiteren bevorzugten Ausführungsform kodiert das dem durch das rekombinante Herstellungsverfahren erzeugte Protein zugrunde liegende Gen für die volle Aminosäuresequenz und eine Transmembranankersequenz.

In einer besonders bevorzugten Ausführungsform umfaßt das rekombinante Herstellungsverfahren folgende Schritte.

Zunächst den Entwurf der zu synthetisierenden DNA-Sequenz auf der Basis des Gens aus *P*. *falciparum* FCB-1, wobei eine DNA-Sequenz mit den z.B. im menschlichen Genom üblichen Codon-Häufigkeiten unter Beibehalten der Aminosäuresequenz des FCB-1-Proteins hergestellt wird.

Hierdurch sollte der AT-Gehalt des Gens reduziert werden, vorzugsweise auf 55%.

Im weiteren Verfahren wird die entworfene Sequenz bspw. in 5 überlappende Regionen eingeteilt, welche jeweils Domänen der natürlichen Prozessierungsprodukte des gp190/MSP1-Proteins aus FCB-1 entsprechen: p83, p31, p36, p30 und p19.

Es werden Desoxyoligonukleotide synthetisiert, die jeweils mindestens die gesamte Länge einer Region abdecken.

Besonders bevorzugt werden die Desoxyoligonukleotide so synthetisiert, daß ihre Sequenz abwechselnd dem "oberen" (5' -3') bzw. dem "unteren" (3' - 5') DNA-Strang entspricht. Die Länge dieser Oligonukleotide ist vorzugsweise durchschnittlich 120 Nukleotide und sie überlappt die benachbarten Sequenzen jeweils um ca. 20 Basen.

In einer möglichen Ausführungsform werden DNA-Sequenzen von etwa doppelter Länge wie die jeweiligen Ausgangsproduke durch asymmetrische PCR hergestellt und zwar so, daß die überschüssigen DNA-Sequenzen, die benachbart sind, jeweils den gegenüberliegenden Strang repräsentieren. Dies führt in einem zweiten

PCR-Amplifikationszyklus zu einem Zweitprodukt, das der Länge von vier ursprünglich eingesetzten Oligonukleotiden (abzüglich der überlapppenden Region) entspricht. Die Überführung dieser Produkte in ein überwiegend aus Einzelstrang-DNA bestehendes Präparat durch asymmetrische PCR mit den endständigen Oligonukleotiden erlaubt in einem weiteren Amplifikationsschritt die Herstellung eines 800 bp langen doppelsträngigen DNA-Fragments in nur 25 PCR-Zyklen.

Auf diese Weise werden direkt die kodierenden Regionen für p19, p30, p36 und p31 synthetisiert und in *E. coli* molekular kloniert. Klone mit fehlerfreier Sequenz wurden entweder direkt oder durch Zusammensetzen fehlerfreier Teilsequenzen erhalten. Die Region, welche das p83 kodiert, wurde durch Fusion aus zwei etwa 1200 bp umfassenden Sequenzen erhalten.

Im weiteren Verlauf des Verfahrens wurden die einzelnen Sequenzen kloniert. Als Expressionsvektoren bieten sich vorzugsweise die Plasmide pDS56, RBSII ("Hochuli, E., Bannwarth, W. Döbeli, H. Gentz, R., and Stüber, D. (1988) Genetic approach to facilitate purification of recombinant proteins with a novel metal chelate adsorbent. Biotechn. 6, 1321-1325"), pBi-5 ("Baron, U., Freundlieb, S., Gossen, M. and Bujard, H. (1995) Corregulation of two gene activities by tetracycline via a bidirectional promoter. Nucl. Acids Res. 23, 3605-3606") und ppTMCS an. Es sind jedoch auch andere Expressionsvektoren denkbar.

Bevorzugte Wirtsorganismen für die Expression sind *E. coli*, besonders bevorzugt der Stamm DH5alphaZ1 (R. Rutz, Dissertation 1996, Universität Heidelberg), HeLa-Zellen, CHO-Zellen, Toxoplasma gondii (Pfefferkorn, E.R. and Pfefferkorn, C.C. 1976, Toxoplasma gondii: Isolation and preliminary characterization of temperature - sensitive mutants. Exp. Parasitol. 39, 365-376) oder Leishmania. Weitere Wirtssysteme könnten z.B. Hefen, Baculoviren oder Adenoviren sein, wobei der Gegenstand der Erfindung nicht auf die genannten Wirtssysteme beschränkt sein sollte.

Es war eine weitere Aufgabe der vorliegenden Erfindung, eine vollständige, zur Expression geeignete DNA-Sequenz des gp190/MSP1-Oberflächenproteins von P. *falciparum* anzugeben.

Diese Aufgabe wird durch die in Anspruch 17 genannte Erfindung gelöst, wobei die Sequenz durch das oben beschriebene rekombinante Herstellungsverfahren erhältlich ist.

In einer bevorzugten Ausführungsform der vorliegenden Erfindung kodiert die zur Expression geeignete DNA-Sequenz für die vollständige Aminosäuresequenz.

In einer anderen bevorzugten Ausführungsform der vorliegenden Erfindung kodiert die zur Expression geeignete DNA-Sequenz für die vollständige Aminosäuresequenz mit Ausnahme des Ankersignals.

In einer weiteren bevorzugten Ausführungsform gemäß der vorliegenden Erfindung kodiert die zur Expression geeignete DNA-Sequenz für die vollständige Aminosäuresequenz mit Ausnahme des Ankersignals und des Signalpeptids. Diese Ausführungsform von gp190/MSP1 kann dadurch gekennzeichnet sein, daß sie am N-Terminus 11 zusätzliche Aminosäuren, davon 6 Histidine, enthält.

Besonders bevorzugt enthält die zur Expression geeignete DNA-Sequenz keine erkennbaren "splice-donor" und "splice-acceptor"-Signale, und sie ist vorzugsweise dadurch gekennzeichnet, daß sie keine größeren GC-reichen Sequenzen enthält, die stabile Haarnadelstrukturen auf RNA-Ebene bewirken könnten.

Vorzugsweise sollten Erkennungssignale für Restriktionsenzyme, welche Sequenzen von sechs und mehr Basenpaaren erkennen, vermieden werden.

In einer bevorzugten Ausführung werden spezifische, d.h. nur einmal im Gen vorkommende Schnittstellen für Restriktionsendonukleasen in Regionen eingeführt, welche die nach der Prozessierung des Proteins entstehenden Domänen trennen.

Besonders bevorzugt sollten an beiden Enden des Gens Sequenzen für Restriktionsendonukleasen vorhanden sein, die im Gen nicht vorkommen.

Weiterhin werden durch die Erfindung Wirtsorganismen zur Verfügung gestellt, die die vollständige Sequenz des gp190/MSP-1 Oberflächenproteins enthalten.

Solche Wirtsorganismen sind vorzugsweise *E*. *coli*, besonders bevorzugt der Stamm DH5alphaZ1, HeLA-Zellen, CHO-Zellen, Toxoplasma gondii oder Leishmania. Die HeLa-Zellen und CHO-Zellen sollten vorzugsweise konstitutiv tTA synthetisieren.

Schließlich stellt die vorliegende Erfindung eine Möglichkeit zur Verfügung, ein nach dem rekombinanten Herstellungsverfahren erzeugtes gp190/MSP1-Oberflächenprotein oder Teile desselben zur aktiven Immunisierung gegen Malaria zu verwenden.

Das hier vorgestellte Syntheseschema erlaubt auch das zweite Allel des gp190/MSP1-Gens herzustellen. Damit wird dem Dimorphismus des Proteins Rechnung getragen. Die Hauptvariabilität des Proteins beruht jedoch auf den Sequenzen von zwei relativ kurzen Blöcken (Block II und IV, Ref. 1), die oligomorph sind. Die vorliegenden Sequenzdaten ermöglichen es, daß mit 6-8 Sequenzkombinationen dieser Blöcke über 95% aller bekannten gp190/MSP1-Sequenzen abgedeckt werden können. Die Synthese dieser Sequenzvarianten läßt sich problemlos anhand der hier vorgestellten Strategien verwirklichen, so daß Varianten sowohl in das K1 als auch in das MAD20-Allel eingebaut werden können. Impfstoffe aus den hierdurch entstehenden Sequenzfamilien können ggf. gegen ein breites Spektrum von Parasiten mit gp190/MSP1-Varianten Schutz verleihen.

Die Herstellung von verschiedenen Impfstofftypen ist möglich:
- Auf der Ebene von Proteinpräparaten, wobei jeweils Mischungen der zwei Familien (K1-Typ, MAD20-Typ mit verschiedenen Varianten der Blöcke II und IV) zur Anwendung kommen können. Verschiedene Träger bzw. Adjuvans-Materialien können zum Einsatz kommen: Aluminiumoxid, Liposomen, Iscoms QSz1 etc.
- Auf der Ebene der Lebendimpfstoffe: (a) virale Träger, insbesondere Vakzinia und Adenoviren; (b) Parasiten als Träger, insbesonder avirulente Formen von Leishmania und Toxoplasma; (c) bakterielle Träger, z. B. Salmonella
- Auf der Ebene der Nukleinsäuren, wobei bspw. für die Gentherapie geeignete Vektoren als Vehikel zum Einbringen der Gene in den Wirt verwendet werden; weiterhin ist das Einbringen von Ribonukleinsäuren, welche das gewünschte Protein kodieren, denkbar.

Eine weitere Möglichkeit der Impfung besteht in der Verwendung eines gemäß dem erfindungsgemäßen rekombinanten Herstellungsverfahren erzeugten gp190/MSP1-Proteins zur Herstellung von monoklonalen Antikörpern, die dann ihrerseits zur passiven Immunisierung gegen Malaria verwendet werden.

Ebenso wird eine Verwendung der gemäß dem rekombinanten Herstellungsverfahren in einem Zwischenschritt entstandenen, dem Protein zugrunde liegenden DNA-Sequenz zur Herstellung einer Vakzine auf Nukleinsäurebasis ermöglicht.

Schließlich betrifft die Erfindung auch ein Verfahren zur Stabilisierung von Gen-Sequenzen, insbesondere von Sequenzen, die keine ausreichende Stabilität in Expressionssystemen zeigen.

Diese Stabilisierung wird erfindungsgemäß dadurch erreicht, daß der AT-Gehalt der Sequenz verringert wird.

Weiterhin wird durch die Erfindung ein stabilisiertes Gen zur Verfügung gestellt, das sich dadurch auszeichnet, daß es einen geringeren AT-Gehalt aufweist. Ein Beispiel für ein solches, stabilisiertes Gen ist das Gen für das gp190/MSP1-Oberflächenprotein gemäß der vorliegenden Erfindung.

Im folgenden soll die Erfindung anhand der Abbildungen und Tabellen sowie einiger Beispiele in einzelnen Ausführungsformen beschrieben werden.

Dabei zeigt:
- Abb. 1:: Schematische Darstellung des gp190/MSP1 Vorläuferproteins aus *P. falciparum* (FCB-1).
- Abb. 2:: Zwei mit nativem gp190/MSP1 aus *P. falciparum* (FCB-1) an Aotus-Affen durchgeführte Impfversuche.
- Abb. 2A:: mit 3 x 60 Mikrogramm gp190/MSP1
- Abb. 2B:: mit 3 x 40 Mikrogramm gp190/MSP1
- Abb. 3A:: Strategie der Synthese des gp190/MSP1-Gens
- Abb. 3B:: Prinzip der PCR-gestützten Total-Synthese
- Abb. 3C:: Totalsequenz des gp190^{S}
- Abb. 3D:: N- und C-Terminus der gp190^{S1}-Variante
- Abb. 4A:: Expressionsvektor pDS56 mit gp190^{S2}-Sequenz
- Abb. 4B:: Gelelektrophorese von gp190^{S2}.
- Abb. 5A:: Expressionsvektor pBi-5 mit gp190^{S1}-Sequenz
- Abb. 5B:: Immunfluoreszenz von HeLa-Zellen
- Abb. 5C:: Elektrophoretische Charakterisierung von aus HeLa-Zellen aufgereinigtem gp190^{S1}
- Abb. 6A:: Expressionsvektor ppT 190 mit gp190-Sequenz
- Abb. 6B:: Immunfluoreszenz der Expression von gp190^{S} in *T. gondii*
- Abb. 6C:: Polyacrylamid-Gelelektrophorese von gp190 aus *T. gondii*

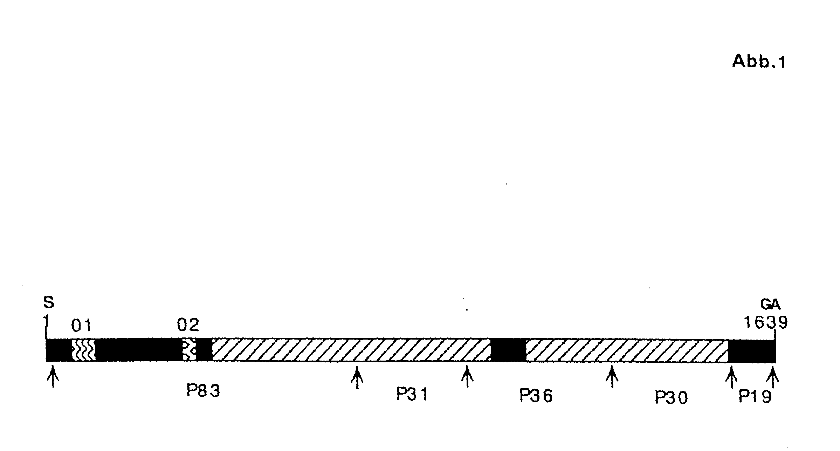

Bei dem in Abb. 1 schematisch dargestellten gp190/MSP1-Vorläuferprotein aus P. *falciparum* (FCB-1) repräsentieren die dunklen Blöcke Regionen, die in allen Stämmen hochkonserviert vorliegen. Die schraffierten Blöcke zeigen die dimorphen Bereiche, welche im Falle des FCB-1 Isolates dem K1-Allel entstammen. O1 und O2 zeigen die oligomorphen Bereiche. S bezeichnet die Signalpeptidsequenz, welche 19 Aminosäuren umfaßt, GA, die C-terminale Region, welche das Signal für die GPI-Verankerung des Proteins in der Membran enthält. Die Pfeile deuten die Prozessierungsstellen an, durch die Proteine p53, p31, p36, p30 und p19 entstehen. Das gp190-Gen kodiert insgesamt 1639 Aminosäuren.

Die weiteren Abbildungen werden im Zusammenhang mit den folgenden Beispielen ausführlicher erläutert.

### BEISPIELE

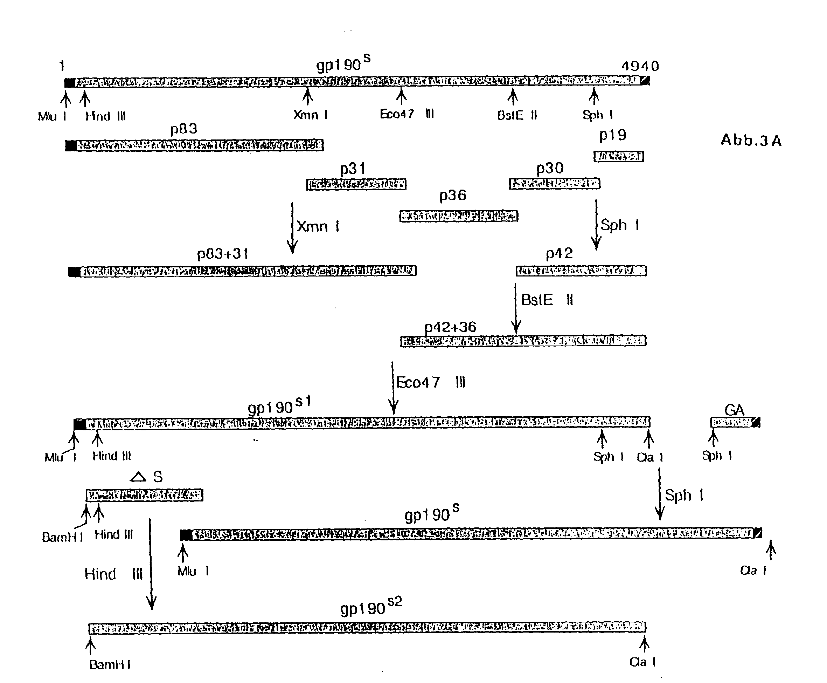

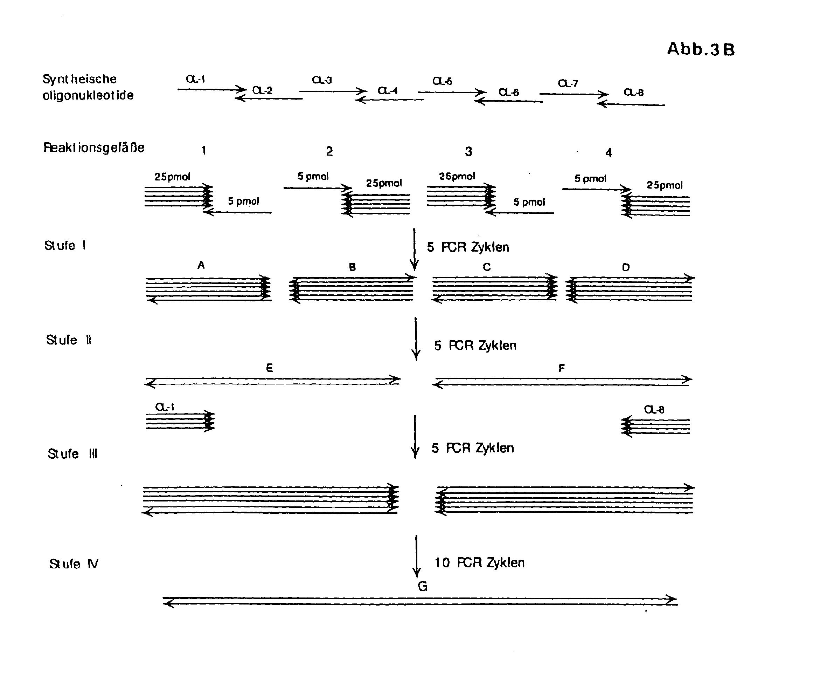

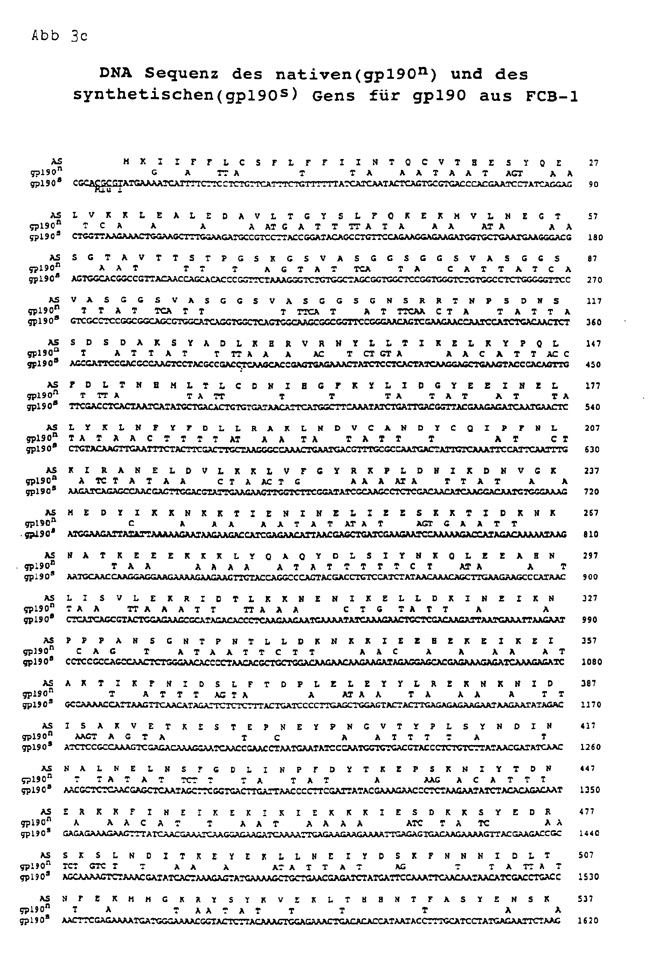

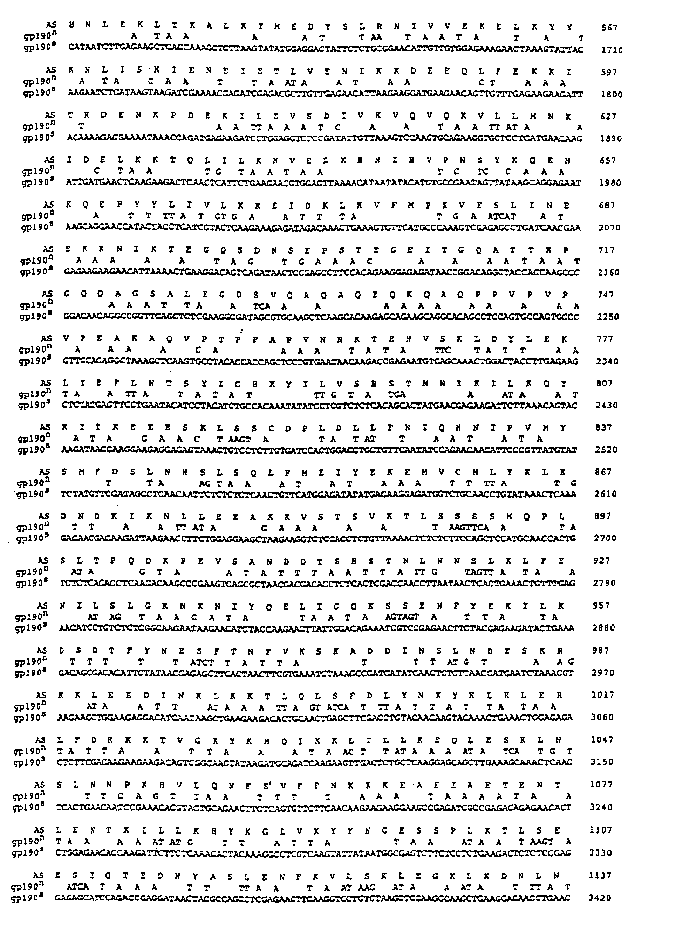

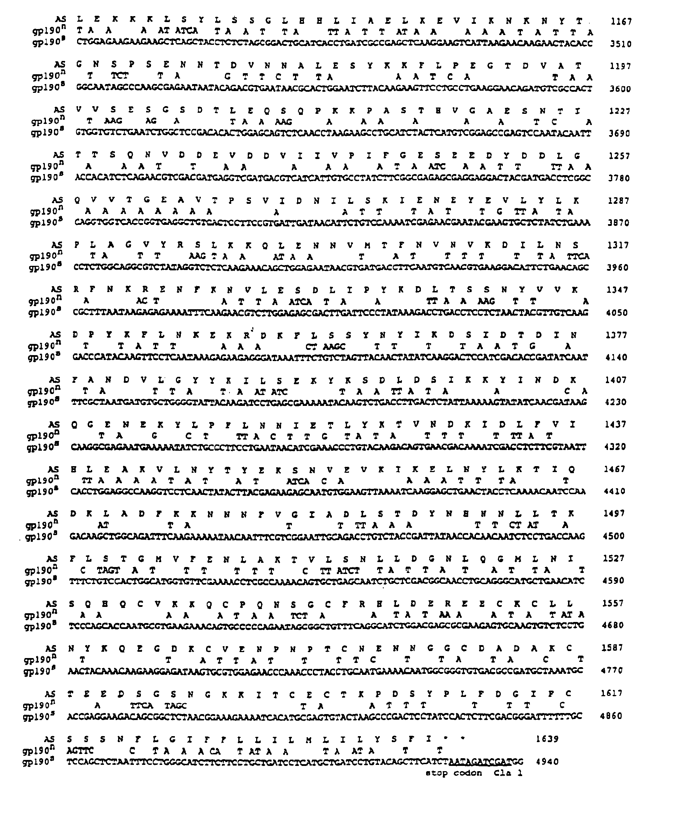

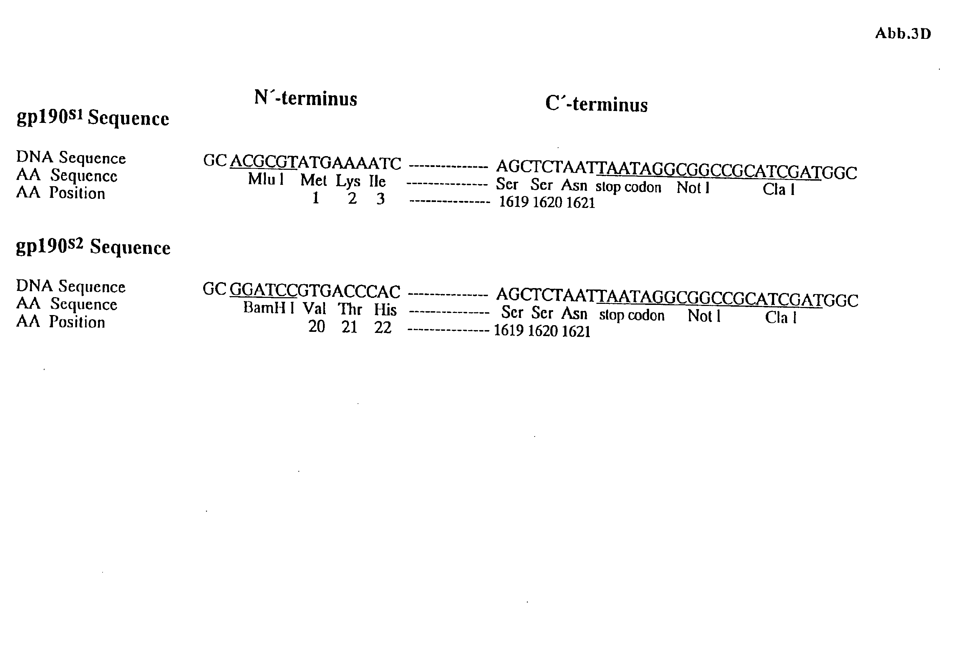

### Beispiel 1: Totalsynthese einer das gp190/MSP1 kodierenden DNA-Sequenz (siehe hierzu Abb. 3)

### A. Strategie der Synthese des gp190/MSP1-Gens (gp190^{S}) (siehe Abb. 3A).

Die Sequenz wurde aufgeteilt in Fragemente, die den Hauptprozessierungsprodukten entsprachen: p83, p31, p36, p30 und p19. In den Übergangsregionen wurden Spaltstellen für Restriktionsendonukleasen (Pfeile in Abb. 3) so eingeplant, daß die Aminosäuresequenz nicht verändert wurde. Alle hier aufgeführten Schnittstellen kommen in der Sequenz nur einmal vor.

Die Fragmente wurden überlappend synthetisiert, so daß die Schnittstellen an den jeweiligen Enden die Verknüpfung zu den benachbarten Fragmenten durch Ligierung möglich machten. Alle Einzelfragmente enthielten zusätzlich an ihrem 5'-Ende eine BamHI-Schnittstelle zur Insertion in Expressionsvektoren. Über Mlul und Clal konnte die Gesamtsequenz kloniert werden. Das hier gezeigte Schema führt zunächst zu einer Sequenz, welche den GPI-Anker nicht ausbilden kann, da C-terminal 18 Aminosäuren fehlen. Die Synthese eines entsprechenden Oligonukleotids, sowie eines über die Sphl-Schnittstelle sich erstreckenden "Primers" führt nach PCR zu dem Fragment GA, das über Sphl und Clal eingesetzt werden konnte, die resultierende Totalsequenz war gp190^{S}. Zur Entfernung der das Signalpeptid kodierenden Sequenz wurden "PCR-Primer" hergestellt, über die das Fragment ΔS synthetisiert wurde. Es erlaubt, über eine BamHI und eine Hindlll-Schnittstelle den N-Terminus so zu verändern, daß das Protein mit Aminosäure Nr. 20 begann. Die Kernsequenz, welche das gp190/MSP1 ohne Signalsequenz und ohne GPI-Anker-Signal kodiert, wurde mit gp 190^{S2} bezeichnet. Deletion des GPI-Ankersignals allein führte zu gp190^{S1}.

### B. Prinzip der PCR-gestützten Totalsynthese (siehe Abb. 3B)

Oligodesoxynukleotide von etwa 120 Nukleotiden Länge wurden abwechselnd vom kodierenden bzw. nichtkodierenden Strang so synthetisiert, daß sie jeweils etwa 20 Basen mit dem benachbarten Fragement überlappten. Das Schema zeigt beispielhaft die Synthese eines ca. 800 bp langen Fragments aus Oligonukleotiden. In der ersten Stufe wurden in 4 Reaktionsgefäßen jeweils 2 Oligonukleotide "asymmetrisch" amplifiziert. Es entstanden 4 etwa 220 bp lange DNA-Populationen, die vorwiegend aus Einzelsträngen bestanden (A, B, C, D). Vereinigung von A und B sowie von C und D und Amplifikation über 5 Zyklen führte zu 2 etwa 400 bp langen doppelsträngigen Produkten. Asymmetrische Amplifikation dieser DNA-Fragmente (Stufe III) ergab Einzelstrangpopulationen, welche nach Vereinigung und Amplifikation (Stufe IV) nach 10 Zyklen das Endprodukt G von ca. 800 bp Länge ergaben. Diese Synthese war ohne Isolierung der Zwischenprodukte und ohne Puffer oder Enzymerneuerung durchführbar, und war nach 3 Stunden beendet. Das Endprodukt wurde elektrophoretisch gereinigt, mit den geeigneten Restriktionsendonukleasen nachgeschnitten und im pBluescript (Stratagene), in dessen Polylinker eine Mlul und eine Clal-Schnittstelle eingesetzt worden waren, in *E*. *coli* kloniert.

### C. Totalsequenz des gp190^{s} (siehe Abb. 3C)

Nach Fusion aller Teilsequenzen (Abb. 3A) in pBluescript wurde die Sequenz des Gens mit der Dideoxymethode verifiziert. Das Leseraster des gp190^{s} hatte eine Länge von 4917 bp (+2 Stopcodons) und kodierte eine Aminosäuresequenz, welche der des gp190/MSP1 aus FCB-1 entspricht (1639 Aminosäuren).

### D. N- und C-Terminus der gp190^{S1}-Variante (siehe Abb. 3D)

Die N-terminale Sequenz, beginnend mit der BamHI-Schnittstelle, zeigte den Übergang bei Aminosäure 20, von der angenommen wird, daß sie nach Abspaltung des Signalpeptids den N-Terminus definiert. Am C-Terminus war die kodierte Sequenz bei Aminosäure 1621 zu Ende. Den Stopcodons folgte die Clal-Schnittstelle.

### Beispiel 2: Expression des gg190^{S2} in E. coli

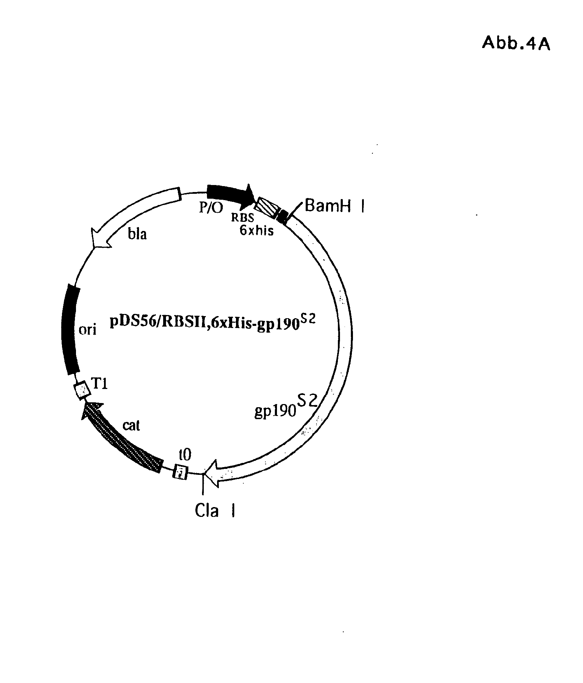

### A. Expressionsvektor (siehe Abb. 4A)

Die gp190^{S2}-Sequenz wurde über die BamHI- und Clal-Schnittstellen in pDS56RBSII eingesetzt. Dadurch wurden 6 Histidine sowie einige aus dem Vektor stammenden Aminosäuren an den N-Terminus fusioniert; dies ergibt folgende N-terminale Sequenz des Leserasters; Met Arg Gly Ser (His)₆ Gly Ser. Durch den Promotor P_{N25lacO-1} stand die Transkription unter lacR/O/IPTG-Kontrolle.

### B. Expression und Aufreinigung von qp190^{S2} (siehe Abb. 4D)

Eine Überführung des Vektors pDS56RBSIIgp190^{S2} in *E. coli* DH5αZ1 und Induktion der Synthese durch IPTG ergab nach elektrophoretischer Auftrennung des Protein-Totalextraktes der Kultur eine deutlich sichtbare Bande in der erwarteten Größe (Pfeil). Die Aufreinigung des Materials durch IMAC und Affinitätschromatographie (Antikörpersäule mit mAK5.2) führte zu einem homogenen Produkt von etwa 190 kD. In der Abb. bedeuten M= Molekulargewichtsstandards; 1 = *E. coli* Proteine vor, 2= nach Induktion mit IPTG für 2 Stunden. 3,4,5 = Fraktionen aus der Elution der mAK-Säule.

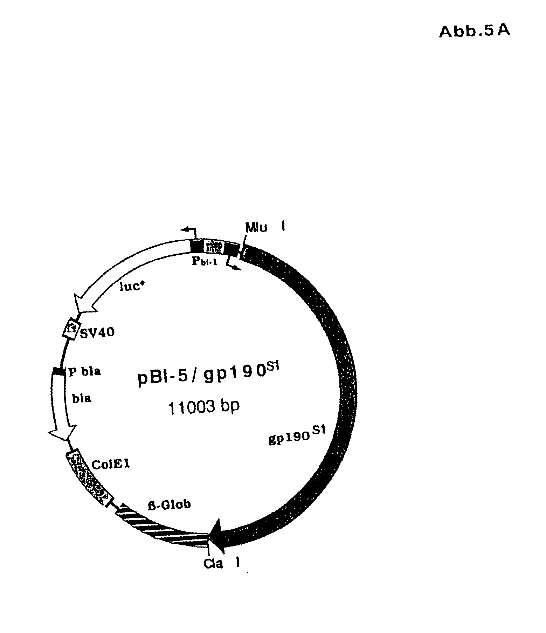

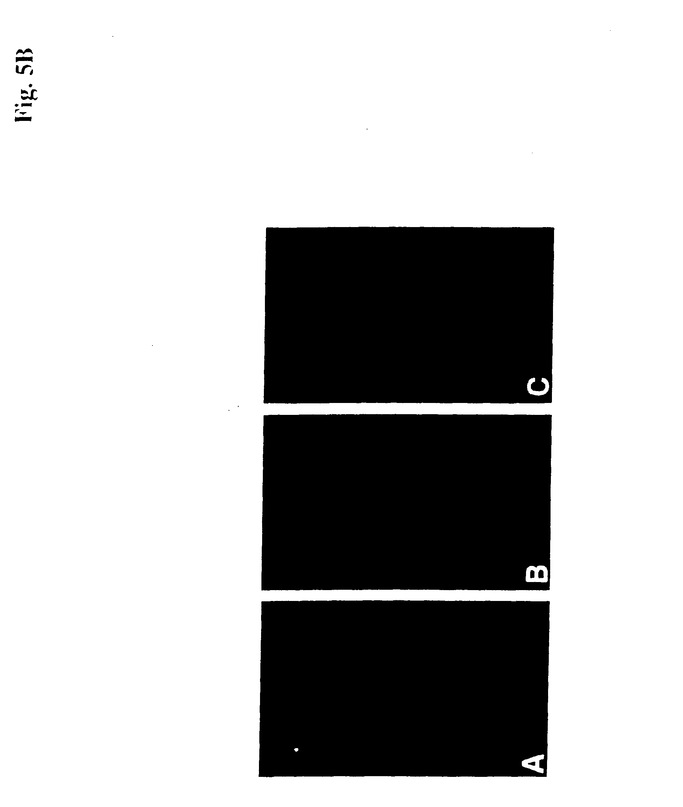

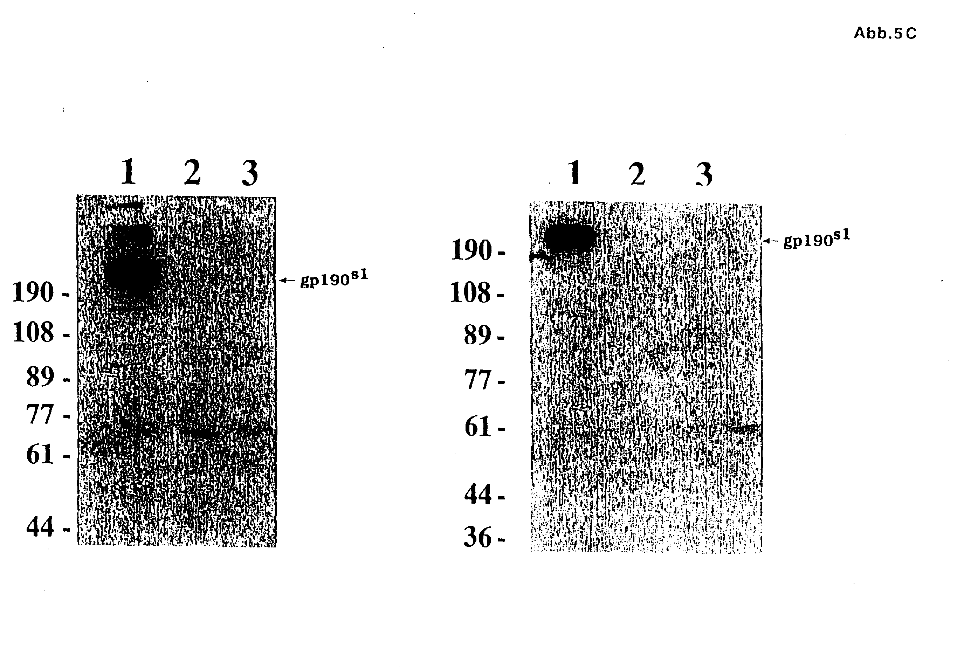

### Beispiel 3: Tetrazyklin-kontrollierte Expression von gp190^{S1} in HeLa- und CHO-Zellen und Isolation des Produktes (siehe auch Abb. 5) bzw. 6c)

A. Die gp190-Sequenz wurde in den Expressionsvektor pBi-5 über die BamHI/Clal-Schnittstellen eingesetzt. Damit stand die Transkription des Gens unter Kontrolle eines bidirektionalen "tTA-responsive"-Promotors und konnte über Tc reguliert werden. Der bidirektionale Promotor initiierte gleichzeitig die Transkription des Indikatorgens Luciferase. Damit ließ sich die Regulation der Expression leicht verfolgen (siehe auch Abb. 5A).

### B. Immunfluoreszenz von HeLa-Zellen, welche Luciferase und gp190^{S1} Tckontrolliert exprimieren.

In HtTA93-9-Zellen, welche die bidirektionale Transkriptionseinheit von (A) enthalten, wurde mit Antikörpern die Produktion von Luciferase (links), gp190^{S1} (Mitte) in Abwesenheit von Tc nachgewiesen. Nach Zugabe von Tc zeigte sich keine nennenswerte Synthese von gp190^{S1}, (wie dargestellt in Abb 5B, rechts).

### C. Elektrophoretische Charkaterisierung von aus HeLa-Zellen aufgereinigtem gp190^{S1}.

HeLa-Zellklon HtTA93-9 sowie CHO-Zellklon CHO27-29 wurden mit oder ohne Tc kultiviert. Durch Elektrophorese aufgetrennte Zellextrakte wurden mittels "Western blot" mit mAK5.2 analysiert (Abb. 5C); links zeigt eine Analyse der CHO-, rechts der HeLa-Zellinie. (1) = Kultur ohne, (2) = Kultur mit Tc, (3) = nicht transfizierte HtTA-1-Zellinie, Molekulargewichtsstandards sind jeweils links angedeutet.

### D. Aufreinigung von in Hela-Zellklon HtTA93-9 synthetisiertem gp190^{S1}

Die präparative Aufzucht der HtTA-93-9-Linie und Induktion der Expression von der gp190^{S1} durch Tc-Entzug erlaubte die Isolierung des Genproduktes über Affinitätschromatographie (mAK5.2-Säule).

Das Coomassie gefärbte Polyacrylamid-Gel (Abb. 6C) zeigte nach Elektrophorese ein Produkt, das aus gp190^{S1} sowie einem weiteren Protein von ca 50 kD bestand. Letzteres war kein Derivat von gp190^{S1}, stammte also aus HeLA-Zellen. Seine gezielte Abtrennung sollte jedoch keine prinzipielle Schwierigkeit darstellen.

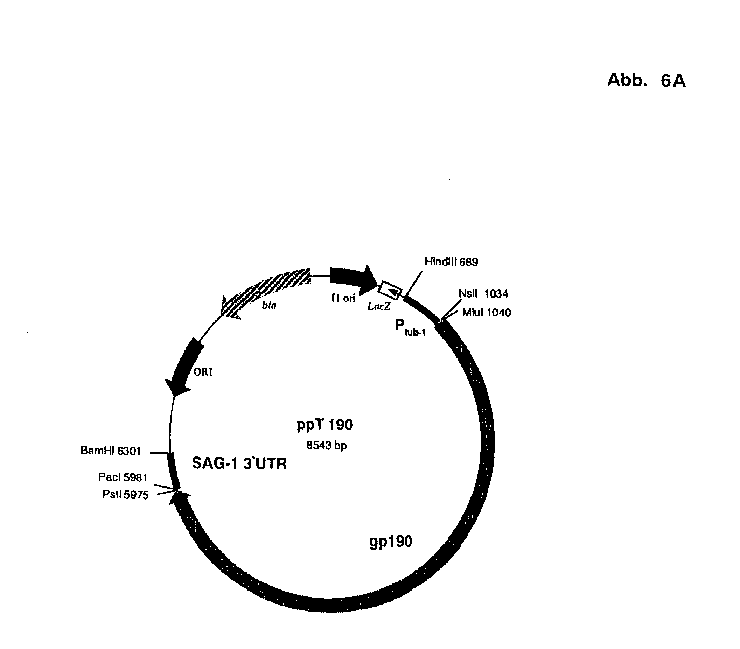

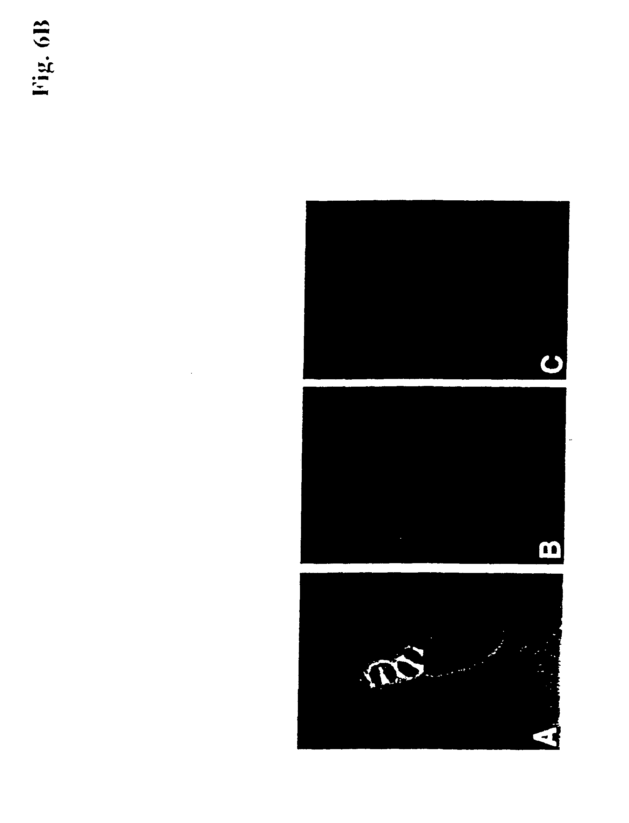

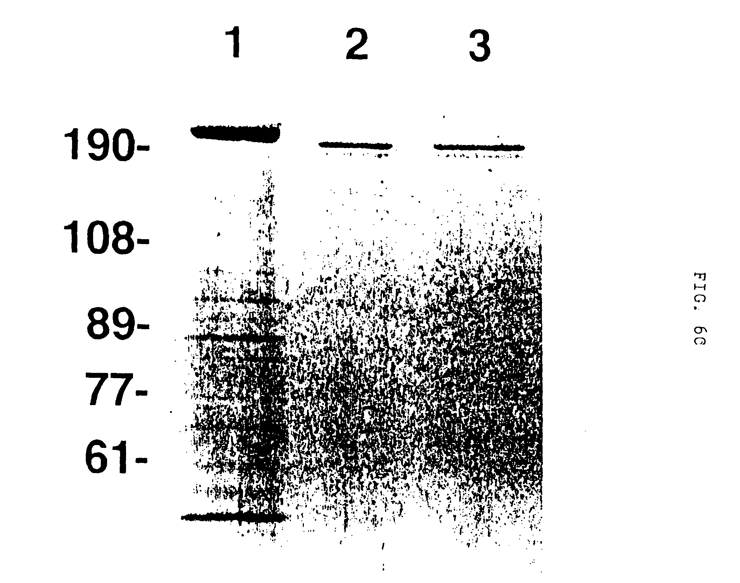

### Beispiel 4: Expression von gp190^{S} in Toxoplasma gondii und Aufreinigung des Produktes (siehe hierzu auch Abb. 6).

A. Die gp190^{s}-Sequenz wurde über MluI/PstI in den Vektor ppT eingesetzt. Damit wurde das Gen unter die Kontrolle des Tubulin-Promotors (P_{tub-1}) von T. gondii gebracht. Die 3' nicht-translatierte Region (VTR) stammte von dem Hauptoberflächenprotein von T. gondii (SAG-1) ab.

### B. Expression von gp190^{S} in T. gondii.

Transfektion von T. gondii mit pTT190 führte zur Isolierung von Parasitenlinien, die konstitutiv gp190^{S} exprimierten. Die Immunfluoreszenz mit mAK5.2 (mittleres Bild) zeigte nicht nur die Expression des Gens, sondern legte auch die Verankerung des Expressionsproduktes an der Oberfläche des Parasiten nahe, da es, wie SAG-1, das gleiche Immunfluoreszenz-Muster erzeugte (rechter Teil der Abb. 6B); links in Abb. 6B ist eine Phasenkontrastaufnahme des mittleren Bildes dargestellt.

### C. Isolierung von qp190^{S} aus T. gondii.

Aus präparativen Mengen von T. gondii (5×10⁹ Parasiten) wurde gp190^{S} mittels Affinitätschromatographie (mAK5.2-Säule) aufgereinigt. Das hochreine Protein besaß das erwartete Molekulargewicht, wie das Coomassie-gefärbte Polyacrylamid-Gel nach Elektrophorese zeigte (2-3 aus Abb. 6C). Bei Nr. (1) Abb. 6C ist gereinigtes gp190^{S1} aus CHO-Zellen dargestellt mit Molekulargewichtsmarkierung an der linken Seite.

### Beispiel 5: Charakterisierung des gp190^{S} mit monoklonalen Antikörpern.

Die Wechselwirkung von 16 monoklonalen Antikörpern mit gp190^{S} aus den verschiedenen heterologen Expressionssystemen wurde durch Immunfluoreszenz (IFA) an P. *falciparum* und T. gondii bzw. durch "Western blot" an den aufgereinigten Proteinen überprüft. Völlige Übereinstimmung wurde gefunden, wenn die beiden Parasiten verglichen wurden (Zahl der + deutet die relative Intensität der Fluoreszenz an). Im Western blot reagieren12mAK's mit gp190^{s} aus *E. coli* und T. gondii. Im Gegensatz dazu binden 3 Antikörper nicht an das aus CHO-Zellen isolierte Material. Antikörper 15 und 16, die Epitope aus dem oligomorphen bzw. dem alternativen Allel (MAD20) erkennen, reagieren nicht. Die Ergebnisse sind in Tab. 1 zusammengefaßt, wobei ND = nicht durchgeführt bedeutet.

### Beispiel 6: Expression des gp190^{S} in heterologen Systemen.

### 1. Expression in E. coli

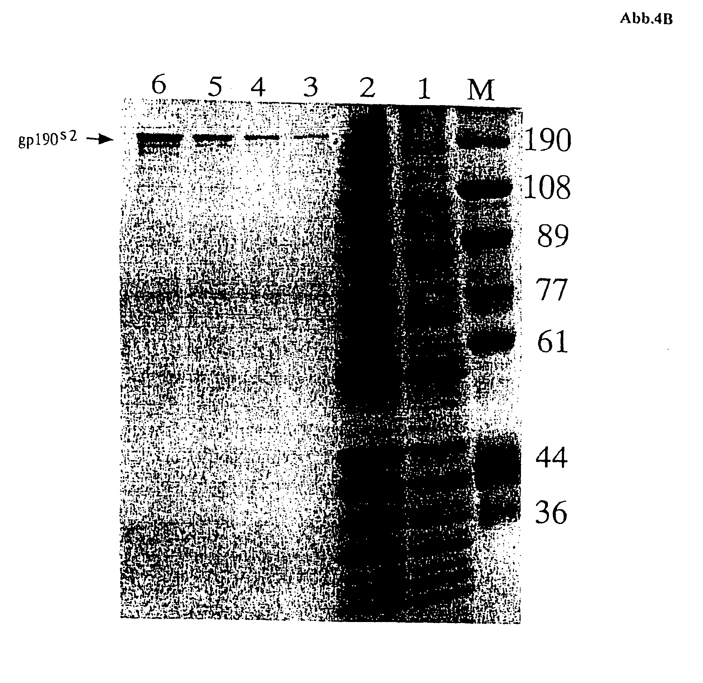

Das gp190^{s2} wurde in den Expressionsvektor, pDS56, RBSII eingesetzt, wo es unter Kontrolle des P_{N25lacO-1}-Promotors stand, der über das lac Operator/Repressor/IPTG-System regulierbar ist (Abb. 4A). Die Überführung des Plasmids in Repressor-produzierende *E. coli*-Zellen, z.B. *E. coli* DH5αZ1 erlaubt es, das gp190^{S2} unter IPTG-Kontrolle zu exprimieren. Das Produkt war aus dem Rohextrakt mittels einer Nickel-Chelatsäule über die durch den Vektor eingebrachte N-terminale (His)₆-Sequenz isolierbar. Eine anschließende Affinitätschromatographie an einer Antikörpersäule führte zu einem hochreinen Präparat. Da der verwendete monoklonale Antikörper (mAk5.2) ein konformationelles Epitop im C-terminalen Bereich erkannte, wird durch diese 2-Stufenreinigung auf intaktes Protein voller Länge, mit korrekter Faltung zumindest am C-Terminus, selektioniert (Abb. 4B).

Das Endprodukt besitzt, im Gegensatz zu dem natürlichen Material, am N-Terminus 11 zusätzliche Aminosäuren, davon 6 Histidine. Es enthält keine N-terminale Signal- und auch keine C-terminale Anker-Sequenz. Die P. falciparum-spezifische Sequenz beginnt mit Aminosäure 20 und endet mit Aminosäure 1621.

### 2. Kontrollierte Expression des gp190^{S1} in HeLa- und CHO-Zellkulturen.

Das gp190^{S1} wurde in den Vektor pBi-5 eingesetzt und damit unter Kontrolle eines durch Tetrazyklin (Tc) regulierbaren Promotors gestellt. Das Tc-kontrollierte System wurde aus 2 Gründen gewählt:
- Es gehört zu den Expressionssystemen, mit denen höchste Ausbeuten in Säugerzellen erreicht werden.
- Nicht-sekretierte Fremdproteine in hoher Konzentration können mit dem Metabolismus der Zellen in negativer Weise interferieren. Die Synthese des gewünschten Produktes wird daher erst nach Aufwachsen der Kultur initiiert.

In dem Konstrukt pBi5-gp190^{S1} wurde ein bidirektionaler Promotor durch Tc-kontrollierten Transkriptionsaktivator (tTA) aktiviert und intitiierte die Transkription sowohl des gp190^{S1} als auch des Luziferase-Indikatorgens. In Anwesenheit von Tc ist der Promotor inaktiv. Die Transkriptionseinheit wurde sowohl in HeLa als auch in CHO-Zellen, welche beide konstitutiv tTA synthetisieren (HtTA-1-Linie (Gossen, M. and Bujard, H. (1992), Tight control of gene expression in mammalian cells by tetracycline-responsive promotors. Proc. Natl. Acad. Sci. USA 89, 5547-5551); CHO-tTA-Linie, unveröffentlicht), überführt. Durch Kotransfektion (Ca²⁺-Phosphat-Methode) mit einem Hygromycin-Resistenz-vermittelnden Markergen wurde auf erfolgreiche chromosomale Integration selektioniert. Hygromycin-resistente Klone wurden dann auf Regulierbarkeit der Expression ≥ Tc untersucht, indem die Luziferaseaktivität als Indikator genutzt wurde. In gut regulierbaren Klonen (Regulationsfaktor ≤ Tc 1000) wurde die gp190 Synthese geprüft. Immunfluoreszenz-Analyse (Abb. 5B) sowie Untersuchungen über "Western blot" (Abb. 5C) erlaubten, von beiden Zelltypen Klone zu identifizieren, welche gp190 unter streng regulierbaren Bedingungen synthetisieren. Von 20 Klonen wurde jeweils der bestregulierbare subkloniert. Die Subklone HtTA93-9 sowie CH027-29 wurden für Kulturen im 10 l Maßstab verwendet. Aus Zellextrakten dieser Kulturen ließ sich intaktes gp190^{S1} mittels Affinitätschromatographie (mAk5.2) isolieren. Das Material ist homogen bis auf eine einzige zelluläre Komponente, die nicht von gp190^{S1} abstammt und die etwa 25% des Präparats ausmacht (Abb. 6C). Sie müßte in einem weiteren Reinigungsschritt entfernt werden.

### 3. Expression des gp190^{s} in Toxoplasma gondii.

Toxoplasma gondii gehört wie P. *falciparum* zu den Apicomplexa und hat daher wahrscheinlich ein dem *P. falciparum* ähnliches Protein-Modifikationssystem. T. gondii läßt sich mit Fremd-DNA transfizieren, die effizient in das Genom integriert wird, außerdem läßt sich T. gondii problemlos in Zellkultur vermehren. Um ein möglichst natives gp190-Produkt zu erhalten, wurde gp190^{S2} so exprimiert, daß das Protein sekretiert und auf der Oberfläche des Parasiten, wie bei *P. falciparum*, über ein GPI-Motiv in der Membran verankert wird. Dazu wurde das gp190^{S2} (Abb. 3A) in das Plasmid ppTMCS (D. Soldati, unveröffentlicht) eingesetzt (Abb. 6A) und damit unter Kontrolle des T. gondii-Tubulin Promotors gestellt.

Dieses Expressionskonstrukt wurde in T. gondii transfiziert. Selektion mit Chloramphenicol führte zu resistenten Klonen, die gp190 synthetisieren, wie durch Immunfluoreszenz nachgewiesen wurde (Abb. 6B). Die immunfluoreszenz mit anti-gp190-Antikörpern war nicht unterscheidbar von einer entsprechenden Anfärbung der Parasiten mittels Antikörper gegen SAG1, dem Hauptoberflächenprotein von T. gondii. Es ist daher davon auszugehen, daß gp190 an der Oberfläche von T. gondii verankert ist. Mehrere T. gondii-Klone (Nr. 3.1 bis 3.4) wurden charakterisiert und für die Produktion von gp190 aufbewahrt. Aus in präparativem Maßstab aufgewachsenen T. gondii-Kulturen (Klon 3.4) wurde gp190 mittels Affinitätschromatographie (mAK5.2.-Säule) isoliert. Analyse im elektrischen Feld zeigte ein homogenes Produkt mit einer Wanderungsgeschwindigkeit, die auf das intakte Protein schließen läßt (Abb. 6C).

### Beispiel 7: Charakterisierung von gp190 Protein aus verschiedenen Expressionssystemen mittels monoklonaler Antikörper.

Ein Satz gp190-spezifischer monoklonaler Antikörper, von denen mehrere konformationelle Epitope erkennen, wurde dazu benutzt, über Immunfluoreszenz die Reaktivität der Antikörper mit *P. falciparum-* bzw. T gondii-Parasiten zu vergleichen. Tabelle 1 zeigt, daß die Reaktivität der 16 Antikörper für beide Parasiten gleich ist. Dies ist ein starker Hinweis darauf, daß in T. gondii weitestgehend "natives" gp190 produziert wird. Der Vergleich der Reaktivität der Antikörper mit Protein aus *E*. *coli*, HeLa- bzw. CHO-Zellen sowie T. gondii zeigt ebenfalls, daß die meisten Antikörper mit den 4 Präparaten reagieren. Insbesondere wird das aus *E*. *coli* isolierte Protein von mehr Antikörpern erkannt als das Produkt aus Säugerzellen. Dies ist wahrscheinlich eine Konsequenz der Glykosylierung in Säugerzellen.

### Beispiel 8: Immunsierung von Aotus lemurinus griseimembra-Affen mit gp190/MSP1 aus P. falciparum (FCB-1).

Zwei unabhängige Immunsierungsexperimente (A, B) wurden durchgeführt. Dazu wurde aus jeweils ca. 2 × 10¹¹ Parasiten unter schonenden Bedingungen einmal 1,0 mg (A) und einmal 0,6 mg hochreines gp190/MSP1 isoliert.

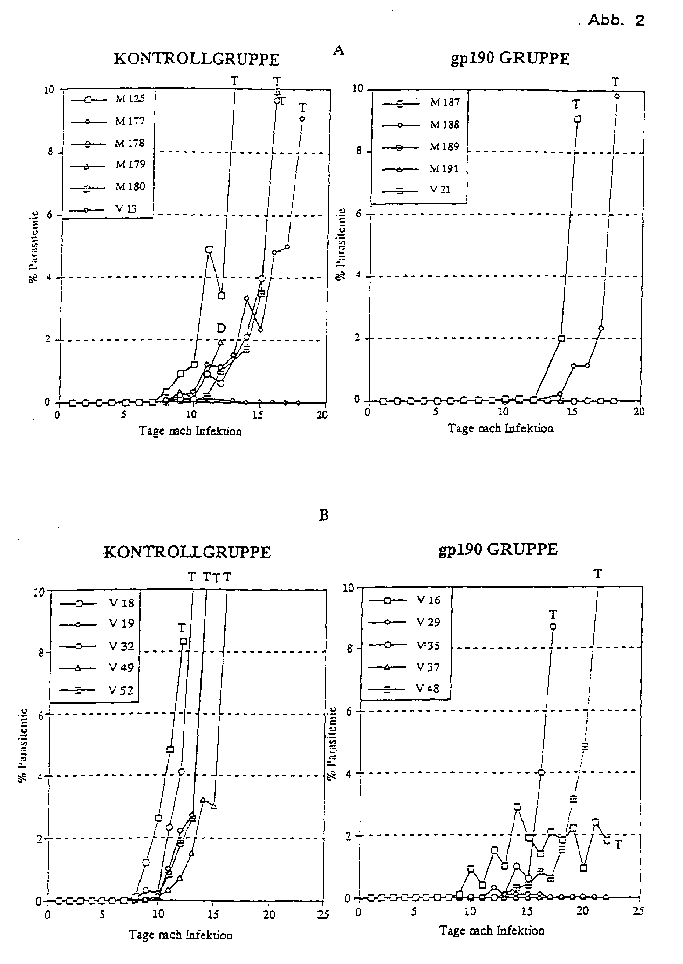

Das Protein wurde zusammen mit Freund'schem Adjuvans (FCA) verabreicht. Die Kontrollgruppe erhielt lediglich FCA. Es wurde dreimal im Abstand von 4 Wochen mit gleicher Menge an Protein bzw. mit Adjuvans immunisiert. Zwei Wochen nach der letzten Immunisierung wurden die Tiere mit jeweils 10⁵ Parasiten (FVO-Stamm) aus einem Donor-Tier infiziert. Die Parasitämie wurde täglich gemessen. Die Ergebnisse sind in Abb. 2 zusammengefaßt. Dabei bedeutet
T: daß die Tiere mit Resochin behandelt wurden
D: ein verstorbenes Tier
Abb. 2A.: die Individuen der geimpften Gruppe erhielten je 3 x 60 Mikrogramm gp190/MSP1
Abb. 2B: die Individuen der geimpften Gruppe erhielten je 3 x 40 Mikrogramm gp190/MSP1

Während in den Kontrollgruppen nur 1/11 Tiere keine Parasitämie entwickelten, waren es in der geimpften Gruppe 6/10. Die vier Tiere aus der geimpften Gruppe, die eine hohe Parasitämie entwickelten, taten dies - im Vergleich zur Kontrollgruppe - mit einer durchschnittlichen Verzögerung von vier Tagen (Überschreiten der 2%-Grenze der Parasitämie).

Diese Experimente zeigten erstmals einen hochsignifikanten Schutz gegen Infektion mit *P. falciparum* durch gp190/MSP1 im Affenmodell. Das erfindungsgemäße Verfahren erlaubt es somit erstmalig, einen wirksamen Impfstoff gegen die Malaria anzugeben.

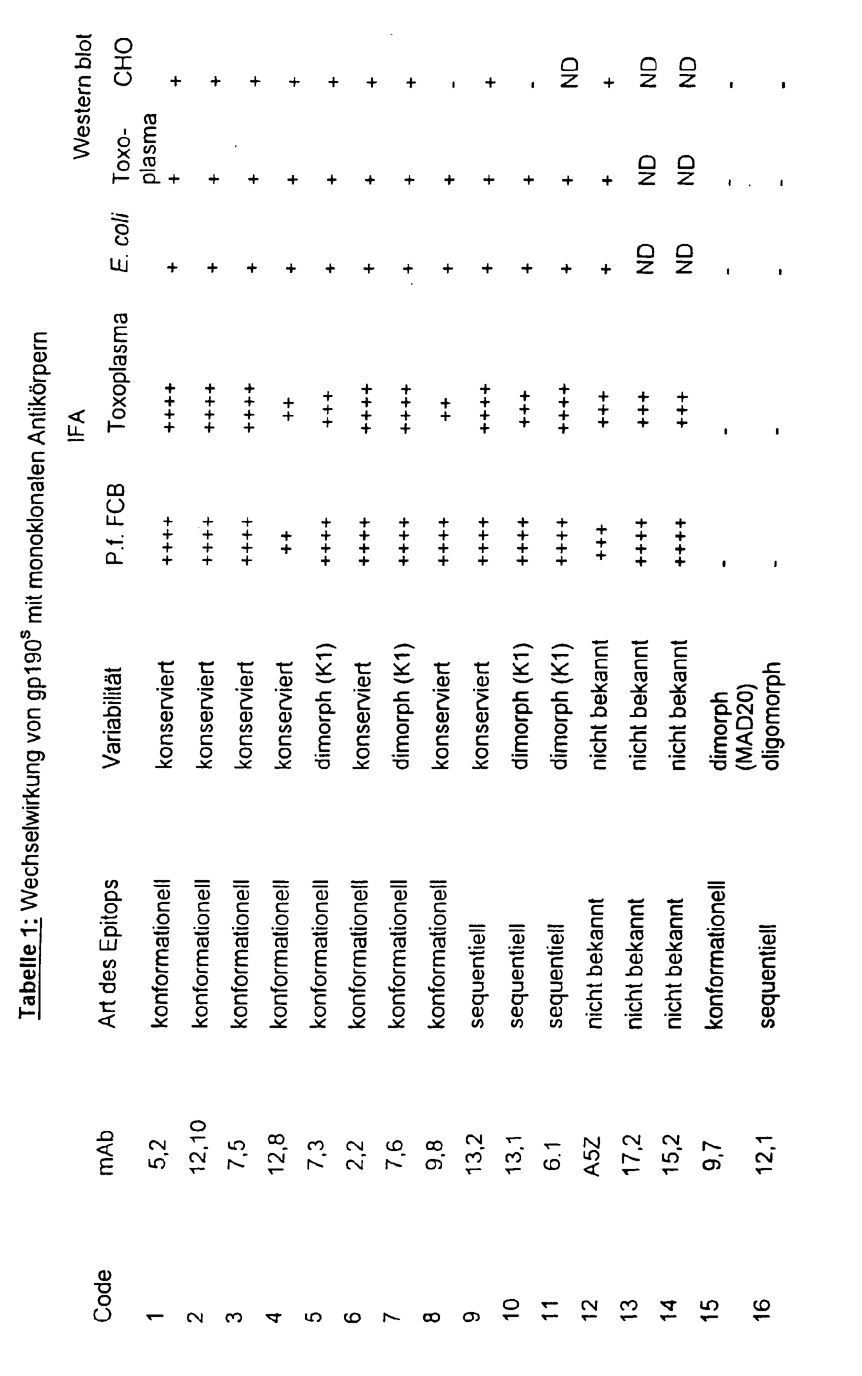

### Annex zu den Anmeldungsunterlagen - nachgereichtes Sequenzprotokoll

## Patentansprüche

1. Wirtsorganismus, der eine vollständige, zur Expression geeignete DNA-Sequenz des gp190/MSP1-Oberflächenproteins von Plasmodium, insbesondere P. *falciparum*, und/oder das vollständige Protein enthält, wobei das gp190/MSP1-Oberflächenprotein durch ein rekombinantes Herstellungsverfahren erhältlich ist, in dem das vollständige Gen für gp190/MSP1 in einem geeigneten System exprimiert wird und der AT-Gehalt der dem Protein zugrunde liegenden, exprimierten DNA-Sequenz gegenüber der natürlich vorkommenden Sequenz erniedrigt ist.

2. Wirtsorganismus gemäß Anspruch 1, **dadurch gekennzeichnet, dass** die zur Expression geeignete DNA-Sequenz nicht für das Ankersignal kodiert.

3. Wirtsorganismus gemäß Anspruch 2, **dadurch gekennzeichnet, dass** die zur Expression geeignete DNA-Sequenz weder für das Ankersignal noch für das Signalpeptid kodiert.

4. Wirtsorganismus gemäß Anspruch 3, **dadurch gekennzeichnet, dass** die zur Expression geeignete DNA-Sequenz eine Sequenz für am N-Terminus vorliegende 11 zusätzliche Aminosäuren, davon 6 Histidine, umfasst.

5. Wirtsorganismus gemäß einem der Ansprüche 1-4, **dadurch gekennzeichnet, dass** die zur Expression geeignete DNA-Sequenz keine erkennbaren "splice donor"- und "splice acceptor"-Signale enthält.

6. Wirtsorganismus gemäß einem der Ansprüche 1-5, **dadurch gekennzeichnet, dass** die zur Expression geeignete DNA-Sequenz keine größeren GC-reichen Sequenzen enthält.

7. Wirtsorganismus gemäß einem der Ansprüche 1-6, **dadurch gekennzeichnet, dass** die zur Expression geeignete DNA-Sequenz keine Erkennungssignale für Restriktionsenzyme, welche Sequenzen von sechs oder mehr Basenpaaren erkennen, enthält.

8. Wirtsorganismus gemäß einem der Ansprüche 1-7, **dadurch gekennzeichnet, dass** die zur Expression geeignete DNA-Sequenz für Erkennungssignale bestimmter Restriktionsnukleasen in Regionen, die die nach der Prozessierung des Proteins entstehenden Domänen trennen, einmal vorkommende Schnittstellen für Restriktionsendonukleasen enthält.

9. Wirtsorganismus gemäß einem der Ansprüche 1-8, **dadurch gekennzeichnet, dass** der Wirtsorganismus E. *coli* ist.

10. Wirtsorganismus gemäß Anspruch 9, **dadurch gekennzeichnet, dass** der E. *coli-*Stamm der Repressor-produzierende E. coli-Stamm DH5alphaZ1 ist.

11. Wirtsorganismus gemäß einem der Ansprüche 1-8, **dadurch gekennzeichnet, dass** der Wirtsorganismus HeLa-Zellen sind.

12. Wirtsorganismus gemäß einem der Ansprüche 1-8, **dadurch gekennzeichnet, dass** der Wirtsorganismus CHO-Zellen sind.

13. Wirtsorganismus gemäß einem der Ansprüche 11 oder 12, **dadurch gekennzeichnet, dass** die Wirtszellen konstitutiv tTA synthetisieren.

14. Wirtsorganismus gemäß einem der Ansprüche 1-8, **dadurch gekennzeichnet, dass** der Wirtsorganismus *Toxoplasma gondii, Leishmania,* Baculoviren, Adenoviren oder Hefen sind.

15. Impfstoff enthaltend ein gp190/MSP1-Oberflächenprotein und/oder eine zur Expression geeignete DNA-Sequenz des gp190/MSP1-Obertlächenproteins und/oder einen Wirtsorganismus gemäß einem der Ansprüche 1-14 und/oder einen Vektor enthaltend eine zur Expression geeignete DNA-Sequenz des gp190/MSP1-Oberflächenproteins, wobei das gp190/MSP1-Oberflächenprotein durch ein rekombinantes Herstellungsverfahren erhältlich ist, in dem das vollständige Gen für gp190/MSP1 in einem geeigneten System exprimiert wird und der AT-Gehalt der dem Protein zugrunde liegenden, exprimierten DNA-Sequenz gegenüber der natürlich vorkommenden Sequenz erniedrigt ist.

16. Impfstoff nach Anspruch 15, **dadurch gekennzeichnet, dass** er weitere Immunität hervorrufende Produkte aus Plasmodium, insbesondere P. *falciparum*, enthält.
